(19) Europäisches Patentamt
European Patent Office
Office européen des brevets

(11) EP 1 700 921 A2

(12) # EUROPEAN PATENT APPLICATION

(43) Date of publication:
**13.09.2006 Bulletin 2006/37**

(51) Int Cl.:
***C12Q 1/26*** *(2006.01)* ***C12N 9/02*** *(2006.01)*

(21) Application number: **06010233.2**

(22) Date of filing: **16.05.2003**

(84) Designated Contracting States:
**DE ES FR GB IT**

(30) Priority: **27.05.2002 EP 02011577**

(62) Document number(s) of the earlier application(s) in accordance with Art. 76 EPC:
**03755104.1 / 1 511 853**

(71) Applicant: **Bayer HealthCare AG**
**51368 Leverkusen (DE)**

(72) Inventor: **The designation of the inventor has not yet been filed**

Remarks:
This application was filed on 18 - 05 - 2006 as a divisional application to the application mentioned under INID code 62.

(54) **Regulation of a human asparagine-hydroxylase**

(57) Reagents that regulate human asparagine-hydroxylase and reagents which bind to human asparagine-hydroxylase gene products can play a role in preventing, ameliorating, or correcting dysfunctions or diseases including, but not limited to cardiovascular disorders, anaemia, cancer, inflammatory diseases, fibrotic disorders, and CNS disorders.

EP 1 700 921 A2

**Description**

## TECHNICAL FIELD OF THE INVENTION

The invention relates to novel human asparagine-hydroxylases. Three novel asparagine-hydroxylases (referred to as ASNH-1 and ASNH-2 and ASNH-3 hereinafter) and their regulation for the treatment of disease are disclosed.

## BACKGROUND OF THE INVENTION

Aspartyl/Asparaginyl-beta-hydroxylase (EC 1.14.11.16) is the only asparagine-hydroxylase cloned from mammalian species and biochemically characterised so far (Korioth F, Gieffers C, and Frey, J. (1994) Gene 150:395-399). The enzyme specifically hydroxylates asparagine or aspartic acid residues at their beta carbon atom in epidermal growth factor (EGF)-like domains of a series of proteins such as coagulation factors (VII, IX, X), complement factors and protein C.

Asparagine-hydroxylation of certain nuclear factors also is implicated in the regulation of oxygen dependent gene expression. The regulation of tissue oxygen supply is of crucial importance for all processes in human life. The level of tissue oxygenation results from the balance between oxygen supply and oxygen consumption. This balance is exactly tuned in the healthy organism but disturbed under many pathological conditions such as pulmonary and cardiovascular diseases, which are characterised by a decrease in oxygen supply, as well as cancer and inflammation, which both are characterised by an increased demand of oxygen within the diseased tissue.

In addition to immediate physiological responses such as vasodilatation, adaptation of heart rate, etc., imbalance of tissue oxygenation is followed by modulation of the transcription rate of a multitude of genes. Among these genes are those that encode for important growth factors and hormones (e.g., vascular endothelial growth factor and erythropoietin) and many metabolic enzymes. The transcriptional modulation leads, for example, to a long lasting adaptation of metabolism, growth, or regression of blood vessels and increased or decreased erythropoiesis.

All oxygen regulated genes have been turned out to be target genes for a distinct family of nuclear transcription factors which were termed hypoxia inducible factors (HIFs). The oxygen regulated genes carry distinct binding sites for HIFs in their regulatory elements (i.e., promoters and enhancers) (Wenger RH, Gassmann M. (1997) Biol Chem. 378 (7):609-16; Semenza GL (1999) Annu Rev Cell Dev Biol. 15:551-78; Zhu H, Bunn HF (1999) Respir Physiol. 115(2): 239-47). In their active form, hypoxia inducible factors consist of an alpha and a beta subunit. While the beta subunit, which was named HIF-1beta or ARNT, is not regulated in response to changes of tissue oxygen, the alpha subunit is unstable under normoxic or hyperoxic conditions. This is due to the rapid degradation of the constitutively translated alpha subunit via the proteasomal pathway after hydroxylation of distinct proline residues and polyubiquitination (Masson N, Willam C, Maxwell PH, Pugh CW, Ratcliffe PJ., EMBO J. 2001 Sep 17;20(18):5197-206.).

Lando et al. (Lando D, Peet DJ, Whelan DA, Gorman JJ, Whitelaw ML, Science. 2002;295(5556):858-61) have shown that beside the regulation of HIFs by ubiquitination a distinct asparagine residue within the carboxyterminal transactivation domain (TADC) of HIF-alpha subunits is important for transcriptional activity of the factor (N803 in human HIF-1 alpha and N851 in human HIF-2 alpha). Under normoxia this asparagine residue is hydroxylated. This hydroxylation inhibits interaction of HIFs with the pleiotropic transcriptional coactivator cbp/p300, thus inactivating HIF. Recently a factor inhibiting HIF (FIH-1) was described which by specific binding to the HIF-alpha TADC suppressed transactivation of reporter genes by HIF (Mahon PC, Hirota K, Semenza GL, Genes Dev. 2001;15(20):2675-86). The biochemical function underlying this suppression has not been resolved. Thus, the specific HIF-asparagine hydroxylase has not been identified so far.

Any HIF-alpha specific asparagine-hydroxylase is a key oxygen sensor for the regulation of oxygen sensitive genes, such as vascular endothelial growth factor, erythropoietin, and iNOS and therefore is of crucial importance for cardiovascular, neoplastic and inflammatory diseases.

## SUMMARY OF THE INVENTION

It is an object of the invention to provide reagents and methods of regulating human asparagine- hydroxylases ASNH-1, ASNH-2, and ASNH-3. This and other objects of the invention are provided by one or more of the embodiments described below.

One embodiment of the invention is an isolated polynucleotide being selected from the group consisting of:

a) a polynucleotide encoding an asparagine-hydroxylase polypeptide comprising an amino acid sequence selected from the group consisting of:

i. amino acid sequences which are at least about 36% identical to the amino acid sequence shown in SEQ ID NO. 2; and

ii. the amino acid shown in SEQ ID NO: 2

iii. amino acid sequences which are at least about 38% identical to the amino acid sequence shown in SEQ ID NO. 4; and

iv. the amino acid shown in SEQ ID NO: 4

v. amino acid sequences which are at least about 28% identical to the amino acid sequence shown in SEQ ID NO. 6;

vi. the amino acid shown in SEQ ID NO: 6.

b) a polynucleotide comprising the sequence of SEQ ID NO: 1, 3 or 5;

c) a polynucleotide which hybridises under stringent conditions to a polynucleotide specified in (a) and (b) encoding a polypeptide exhibiting the biological activity of an asparagine-hydroxylase polypeptide;

d) a polynucleotide the sequence of which deviates from the polynucleotide sequences specified in (a) to (c) due to the degeneration of the genetic code encoding a polypeptide exhibiting the biological activity of an asparagine-hydroxylase polypeptide; and

e) a polynucleotide which represents a fragment, derivative or allelic variation of a polynucleotide sequence specified in (a) to (d) encoding a polypeptide exhibiting the biological activity of an asparagine-hydroxylase polypeptide.

Another embodiment of the invention is an expression vector containing a polynucleotide that encodes a asparagine-hydroxylase or a polypeptide exhibiting the biological activity of an a human asparagine-hydroxylase.

Yet another embodiment of the invention is a host cell containing an expression vector. The expression vector contains a polynucleotide that encodes a human asparagine-hydroxylase or a polypeptide exhibiting the biological activity of an a human asparagine-hydroxylase.

Another embodiment of the invention is a substantially purified polypeptide exhibiting the biological activity of an human asparagine-hydroxylase.

Still another embodiment of the invention is a method for producing an isolated polypeptide exhibiting the biological activity of an human asparagine-hydroxylase. A host cell containing an expression vector is cultured under conditions suitable for expression of a polypeptide encoded by a polynucleotide contained in the expression vector. The polynucleotide encodes a human asparagine-hydroxylase or a polypeptide exhibiting the biological activity of an a human asparagine-hydroxylase. The polypeptide is recovered from the host cell culture.

Another embodiment of the invention is a method for detection of polynucleotides encoding a human asparagine-hydroxylase or a polypeptide exhibiting the biological activity of an a human asparagine-hydroxylase in a biological sample. A polynucleotide encoding a human asparagine-hydroxylase or a polypeptide exhibiting the biological activity of an a human asparagine-hydroxylase is hybridized to nucleic acid material of a biological sample, thereby forming a hybridization complex. The hybridization complex is detected.

Even another embodiment of the invention is a method for the detection of a polynucleotide encoding a human asparagine-hydroxylase or a polypeptide exhibiting the biological activity of an asparagine-hydroxylase. A biological sample is contacted with a reagent which specifically interacts with the polynucleotide or protein. The interaction is detected.

Another embodiment of the invention is a diagnostic kit for detecting a polynucleotide encoding a human asparagine-hydroxylase or a polypeptide exhibiting the biological activity of an human asparagine-hydroxylase in a biological sample.

Even another embodiment of the invention is a method of screening for agents that can regulate the activity of a human asparagine-hydroxylase. A test compound is contacted with a human asparagine-hydroxylase polypeptide or a polypeptide exhibiting the biological activity of an asparagine-hydroxylase. Binding of the test compound to the polypeptide is detected. A test compound that binds to the polypeptide is identified as a potential therapeutic agent for regulating the activity of human asparagine-hydroxylase.

Even another embodiment of the invention is a method of screening for agents that regulate the activity of a human asparagine-hydroxylase. A test compound is contacted with a human asparagine-hydroxylase polypeptide or a polypeptide exhibiting the biological activity of an asparagine-hydroxylase. A asparagine-hydroxylase activity of the polypeptide is detected. A test compound that increases the asparagine-hydroxylase activity is identified as a potential therapeutic agent for increasing the activity of the human asparagine-hydroxylase. A test compound that decreases the asparagine-hydroxylase activity of the polypeptide is identified as a potential therapeutic agent for decreasing the activity of the asparagine-hydroxylase.

Still another embodiment of the invention is a method of screening for agents that regulate the activity of a human asparagine-hydroxylase. A test compound is contacted with a polynucleotide encoding a human asparagine-hydroxylase or a polypeptide exhibiting the biological activity of an asparagine-hydroxylase. Binding of the test compound to the polynucleotide is detected. A test compound that binds to the polynucleotide is identified as a potential therapeutic agent for regulating the activity of human asparagine-hydroxylase.

Yet another embodiment of the invention is a method of regulating the activity of human asparagine-hydroxylase. A cell is contacted with a reagent that specifically binds to a polynucleotide encoding a human asparagine-hydroxylase or a polypeptide exhibiting the biological activity of an a human asparagine-hydroxylase. The activity of human asparagine-hydroxylase is thereby reduced.

A further embodiment of the invention is a purified reagent that regulates the activity of a human asparagine-hydroxylase polypeptide or polynucleotide or a polypeptide exhibiting the biological activity of an asparagine-hydroxylase.

Another embodiment of the invention is the use of a reagent that regulates the activity of a human asparagine-hydroxylase polypeptide or polynucleotide or a polypeptide exhibiting the biological activity of an asparagine-hydroxylase in the preparation of a medicament for the treatment of carcinomas and leukemias, anaemia, chronic inflammatory diseases, including but not limited to rheumatoid arthritis, and cardiovascular diseases.

Another embodiment of the invention is a pharmaceutical composition comprising a reagent that regulates the activity of a human asparagine-hydroxylase and a pharmaceutically acceptable carrier.

Another embodiment of the invention is a pharmaceutical composition comprising an expression vector comprising a polynucleotide encoding a human asparagine-hydroxylase and a pharmaceutically acceptable carrier.

Still another embodiment of the invention is a method for treating disease. An effective amount of a pharmaceutical composition comprising a reagent that regulates the activity of a human asparagine-hydroxylase or a polypeptide exhibiting the biological activity of an asparagine-hydroxylase or an expression vector comprising a polynucleotide encoding a human asparagine-hydroxylase and a pharmaceutically acceptable carrier is administered to a subject in need of such treatment.

The invention thus provides novel human asparagine-hydroxylases that can be used to identify test compounds that may act, for example, as activators or inhibitors at the enzyme's active site. Human asparagine-hydroxylase and fragments thereof also are useful in raising specific antibodies that can block the enzyme and effectively reduce its activity.

## BRIEF DESCRIPTION OF THE DRAWINGS

FIG. 1 shows the activity of GAL4 HIF-I alpha TADC and HIF-2 alpha TADC and the corresponding asparagine/alanine mutants in the presence of ASNH-3.

FIG. 2 shows the expression of ASNH-1 in human tissues as analysed by quantitative PCR techniques (TaqMan).

FIG. 3 shows the expression of ASNH-2 in human tissues as analysed by quantitative PCR techniques (TaqMan).

FIG. 4 shows the expression of ASNH-3 in human tissues as analised by quantitative PCR techniques (TaqMan).

## DETAILED DESCRIPTION OF THE INVENTION

The present invention is based on the discovery of three novel human asparagine hydroxylases (referred to as ASNH-1, ASNH-2 and ASNH-3 in the following). The only human asparagine hydroxylase described in the literature (EC 1.14.11.16) is a dioxygenase which catalyses the hydroxylation of L-asparagine and/or aspartate within epidermal growth factor-like domains of various polypeptides.

Hydroxylation of asparagine by asparagine hydroxylase requires 2-oxoglutarate, $O_2$, $Fe^{2+}$ and ascorbate. The following is an example of an asparagine-hydroxylase reaction:

$$\text{Peptide L-asparagine} + \text{2-oxoglutarate} + O_2 \longleftrightarrow \text{peptide 3-hydroxy L-asparagine} + \text{succinate} + CO_2$$

ASNH-1 to 3 are novel HIF asparagine-hydroxylases that catalyse hydroxylation of the critical asparagine residues within the TADC of HIFs, and therefore are involved in the transcriptional inactivation of HIFs under normoxia. The activity was functionally tested. (for experimental description see Example 5, for experimental data see Fig. 1).

Human asparagine hydroxylase ASNH-1 comprises the amino acid sequence shown in SEQ ID NO: 2. A coding sequence for human asparagine hydroxylase is shown in SEQ ID NO: 1.

Human asparagine hydroxylase ASNH-2 comprises the amino acid sequence shown in SEQ ID NO: 4. A coding sequence for human asparagine hydroxylase is shown in SEQ ID NO: 3.

Human asparagine hydroxylase ASNH-3 comprises the amino acid sequence shown in SEQ ID NO: 6. A coding sequence for human asparagine hydroxylase is shown in SEQ ID NO: 5.

Human asparagine hydroxylases ASNH-1, ASNH-2 and ASNH-3 of the invention are useful in therapeutic methods to treat disorders such as cancer, cardiovascular disorders, anaemia, CNS disorders, inflammatory diseases, and fibrotic disorders. ASNH-1, ASNH-2 and ASNH-3 also can be used to screen for human asparagine hydroxylase regulators (activators and inhibitors). Regulators can be used in the preparation of a medicament for the treatment of said diseases.

*Polypeptides*

Human asparagine-hydroxylase polypeptides according to the invention comprise at least 6, 10, 15, 20, 25, 50, 75, 100, 125, 150, 175, 200, 225, 250, 275, 300, 325, 350, or 369 contiguous amino acids selected from the amino acid sequence shown in SEQ ID NO: 2 or a biologically active variant thereof, as defined below, or at least 6, 10, 15, 20, 25, 50, 75, 100, 125, 150, 175, 200, 225, 250, 275, 300, 325, 350, or 373 contiguous amino acids selected from the amino acid sequence shown in SEQ ID NO: 4 or a biologically active variant thereof, as defined below, or at least 6, 10, 15, 20, 25, 50, 75, 100, 125, 150, 175, 200, 225, 250, 275, 300, 325, or 349 contiguous amino acids selected from the amino acid sequence shown in SEQ ID NO: 6 or a biologically active variant thereof, as defined below. An asparagine-hydroxylase polypeptide of the invention therefore can be a portion of a asparagine-hydroxylase protein, a full-length asparagine-hydroxylase protein, or a fusion protein comprising all or a portion of a asparagine-hydroxylase protein.

*Biologically Active Variants*

Human asparagine-hydroxylase polypeptide variants that are biologically active, e.g., retain a asparagine-hydroxylase activity, also are asparagine-hydroxylase polypeptides. Preferably, naturally or non-naturally occurring asparagine-hydroxylase polypeptide variants have amino acid sequences which are at least about 28, 36, 38, 40, 45, 50, 55, 60, 65, or 70, preferably about 75, 80, 85, 90, 96, 96, 98, or 99% identical to the amino acid sequence shown in SEQ ID NO: 2, 4 or 6 or a fragment thereof. Percent identity between a putative asparagine-hydroxylase polypeptide variant and an amino acid sequence of SEQ ID NO: 2, 4 or 6 is determined by conventional methods. See, for example, Altschul et al., Bull. Math. Bio. 48:603 (1986), and Henikoff & Henikoff, Proc. Natl. Acad. Sci. USA 89:10915 (1992). Briefly, two amino acid sequences are aligned to optimize the alignment scores using a gap opening penalty of 10, a gap extension penalty of 1, and the "BLOSUM62" scoring matrix of Henikoff & Henikoff, 1992.

Those skilled in the art appreciate that there are many established algorithms available to align two amino acid sequences. The "FASTA" similarity search algorithm of Pearson & Lipman is a suitable protein alignment method for examining the level of identity shared by an amino acid sequence disclosed herein and the amino acid sequence of a putative variant. The FASTA algorithm is described by Pearson & Lipman, Proc. Nat'l Acad. Sci. USA 85:2444(1988), and by Pearson, Meth. Enzymol. 183:63 (1990). Briefly, FASTA first characterizes sequence similarity by identifying regions shared by the query sequence (e.g., SEQ ID NO: 2, 4 or 6) and a test sequence that have either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff ' value (calculated by a predetermined formula based upon the length of the sequence the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm (Needleman & Wunsch, J Mol. Biol.48:444 (1970); Sellers, SIAM J. Appl. Math.26:787 (1974)), which allows for amino acid insertions and deletions. Preferred parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, Meth. Enzymol. 183:63 (1990).

FASTA can also be used to determine the sequence identity of nucleic acid molecules using a ratio as disclosed above. For nucleotide sequence comparisons, the ktup value can range between one to six, preferably from three to six, most preferably three, with other parameters set as default.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a asparagine-hydroxylase polypeptide can be found using computer programs well known in the art, such as DNASTAR software. Whether an amino acid change results in a biologically active asparagine-hydroxylase polypeptide can readily be determined by assaying for asparagine-hydroxylase activity, as described for example, in Kivirikko, K. I., Myllylä, T. (1982) Methods Enzymol. 82, 245-304, or Cuncliffe, C. J., Franklin, T. J., Gaskell, R. M. (1986) Biochem. J. 240, 617-619.

*Fusion Proteins*

Fusion proteins are useful for generating antibodies against asparagine-hydroxylase polypeptide amino acid sequences and for use in various assay systems. For example, fusion proteins can be used to identify proteins that interact with portions of a asparagine-hydroxylase polypeptide. Protein affinity chromatography or library-based assays for protein-protein interactions, such as the yeast two-hybrid or phage display systems, can be used for this purpose. Such methods are well known in the art and also can be used as drug screens.

A asparagine-hydroxylase polypeptide fusion protein comprises two polypeptide segments fused together by means of a peptide bond. The first polypeptide segment comprises at least 6, 10, 15, 20, 25, 50, 75, 100, 125, 150, 175, 200, 225, 250, 275, 300, 325, 350, or 369 contiguous amino acids selected from the amino acid sequence shown in SEQ ID NO: 2 or a biologically active variant thereof, as defined above, or at least 6, 10, 15, 20, 25, 50, 75, 100, 125, 150, 175, 200, 225, 250, 275, 300, 325, 350, or 373 contiguous amino acids selected from the amino acid sequence shown in SEQ ID NO: 4 or a biologically active variant thereof, as defined above, or at least 6, 10, 15, 20, 25, 50, 75, 100, 125, 150, 175, 200, 225, 250, 275, 300, 325, or 349 contiguous amino acids selected from the amino acid sequence shown in SEQ ID NO: 6 or a biologically active variant thereof, as defined above.. The first polypeptide segment also can comprise full-length asparagine-hydroxylase protein.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the asparagine-hydroxylase polypeptide-encoding sequence and the heterologous protein sequence, so that the asparagine-hydroxylase polypeptide can be cleaved and purified away from the heterologous moiety.

A fusion protein can be synthesised chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from SEQ ID NO: 1, 3 or 5 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KTTS).

*Identification of Species Homologs*

Species homologs of human asparagine-hydroxylase polypeptides can be obtained using asparagine-hydroxylase polypeptide polynucleotides (described below) to make suitable probes or primers for screening cDNA expression libraries from other species, such as mice, monkeys, or yeast, identifying cDNAs which encode homologs of asparagine-hydroxylase polypeptides, and expressing the cDNAs as is known in the art.

*Polynucleotides*

A asparagine-hydroxylase polynucleotide can be single- or double-stranded and comprises a coding sequence or the complement of a coding sequence for a asparagine-hydroxylase polypeptide having either an amino acid sequence shown in SEQ ID NO: 2, 4 or 6 or a biologically active variant thereof. A coding sequence for SEQ ID NO: 2 is shown in SEQ ID NO: 1; a coding sequence for SEQ ID NO: 4 is shown in SEQ ID NO: 3; a coding sequence for SEQ ID NO: 6 is shown in SEQ ID NO: 5.

Degenerate nucleotide sequences encoding human asparagine-hydroxylase polypeptides, as well as homologous nucleotide sequences which are at least about 50, 55, 60, 65, 70, preferably about 75, 90, 96, 98, or 99% identical to the nucleotide sequences shown in SEQ ID NO: 1, 3 or 5 or their complements also are asparagine-hydroxylase polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologues, and variants of asparagine-hydroxylase polynucleotides that encode biologically active asparagine-hydroxylase polypeptides also are asparagine-hydroxylase polynucleotides. Polynucleotide fragments comprising at least 8, 9, 10, 11, 12, 15, 20, or 25 contiguous nucleotides of SEQ ID NO: 1, 3 or 5 or their complements also are asparagine-hydroxylase polynucleotides. These fragments can be used, for example, as hybridisation probes or as antisense oligonucleotides.

*Identification of Polynucleotide Variants and Homologues*

Variants and homologues of the asparagine-hydroxylase polynucleotides described above also are asparagine-hydroxylase polynucleotides. Typically, homologous asparagine-hydroxylase polynucleotide sequences can be identified by hybridisation of candidate polynucleotides to known asparagine-hydroxylase polynucleotides under stringent conditions, as is known in the art. For example, using the following wash conditions-2X SSC (0.3 M NaCl, 0.03 M sodium citrate, pH 7.0), 0.1% SDS, room temperature twice, 30 minutes each; then 2X SSC, 0.1% SDS, 50°C once, 30 minutes; then 2X SSC, room temperature twice, 10 minutes each--homologous sequences can be identified which contain at most about 25-30% basepair mismatches. More preferably, homologous nucleic acid strands contain 15-25% basepair mismatches, even more preferably 5-15% basepair mismatches.

Species homologues of the asparagine-hydroxylase polynucleotides disclosed herein also can be identified by making suitable probes or primers and screening cDNA expression libraries from other species, such as mice, monkeys, or yeast. Human variants of asparagine-hydroxylase polynucleotides can be identified, for example, by screening human cDNA expression libraries. It is well known that the $T_m$ of a double-stranded DNA decreases by 1-1.5°C with every 1% decrease in homology (Bonner et al., J. Mol. Biol. 81, 123 (1973). Variants of human asparagine-hydroxylase polynucleotides or asparagine-hydroxylase polynucleotides of other species can therefore be identified by hybridising a putative homologous asparagine-hydroxylase polynucleotide with a polynucleotide having a nucleotide sequence of SEQ ID NO: 1, 3 or 5 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridise to asparagine-hydroxylase polynucleotides or their complements following stringent hybridisation and/or wash conditions also are asparagine-hydroxylase polynucleotides. Stringent wash conditions are well known and understood in the art and are disclosed, for example, in Sambrook et al., MOLECULAR CLONING: A LABORATORY MANUAL, 2d ed., 1989, at pages 9.50-9.51.

Typically, for stringent hybridisation conditions a combination of temperature and salt concentration should be chosen that is approximately 12-20°C below the calculated $T_m$ of the hybrid under study. The $T_m$ of a hybrid between a asparagine-hydroxylase polynucleotide having a nucleotide sequence shown in SEQ ID NO: 1, 3 or 5 or the complement thereof and a polynucleotide sequence which is at least about 50, preferably about 75, 90, 96, or 98% identical to one of those nucleotide sequences can be calculated, for example, using the equation of Bolton and McCarthy, Proc. Natl. Acad. Sci. U.S.A. 48, 1390 (1962):

$$T_m = 81.5^{\circ}C - 16.6(\log_{10}[Na^+]) + 0.41(\%G + C) - 0.63(\%formamide) - 600/l,$$

where $l$ = the length of the hybrid in basepairs.

Stringent wash conditions include, for example, 4X SSC at 65°C, or 50% formamide, 4X SSC at 42°C, or 0.5X SSC, 0.1% SDS at 65°C. Highly stringent wash conditions include, for example, 0.2X SSC at 65°C.

*Preparation of Polynucleotides*

A asparagine-hydroxylase polynucleotide can be isolated free of other cellular components such as membrane components, proteins, and lipids. Polynucleotides can be made by a cell and isolated using standard nucleic acid purification techniques, or synthesised using an amplification technique, such as the polymerase chain reaction (PCR), or by using an automatic synthesiser. Methods for isolating polynucleotides are routine and are known in the art. Any such technique for obtaining a polynucleotide can be used to obtain isolated asparagine-hydroxylase polynucleotides. For example, restriction enzymes and probes can be used to isolate polynucleotide fragments, which comprise asparagine-hydroxylase nucleotide sequences. Isolated polynucleotides are in preparations that are free or at least 70, 80, or 90% free of other molecules.

Human asparagine-hydroxylase cDNA molecules can be made with standard molecular biology techniques, using asparagine-hydroxylase mRNA as a template. Human asparagine-hydroxylase cDNA molecules can thereafter be replicated using molecular biology techniques known in the art and disclosed in manuals such as Sambrook *et al.* (1989). An amplification technique, such as PCR, can be used to obtain additional copies of polynucleotides of the invention, using either human genomic DNA or cDNA as a template.

Alternatively, synthetic chemistry techniques can be used to synthesise asparagine-hydroxylase polynucleotides. The degeneracy of the genetic code allows alternate nucleotide sequences to be synthesised which will encode a asparagine-hydroxylase polypeptide having, for example, an amino acid sequence shown in SEQ ID NO: 2, 4 6 or a biologically active variant thereof.

## *Extending Polynucleotides*

Various PCR-based methods can be used to extend the nucleic acid sequences disclosed herein to detect upstream sequences such as promoters and regulatory elements. For example, restriction-site PCR uses universal primers to retrieve unknown sequence adjacent to a known locus (Sarkar, PCR Methods Applic. 2, 318-322, 1993). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (Triglia et al., Nucleic Acids Res. 16, 8186, 1988). Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (Lagerstrom et al., PCR Methods Applic. 1, 111-119, 1991). In this method, multiple restriction enzyme digestions and ligations also can be used to place an engineered double-stranded sequence into an unknown fragment of the DNA molecule before performing PCR.

Another method which can be used to retrieve unknown sequences is that of Parker et al., Nucleic Acids Res. 19, 3055-3060, 1991). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analise the size or confirm the nucleotide sequence of PCR or sequencing products. For example, capillary sequencing can employ flowable polymers for electrophoretic separation, four different fluorescent dyes (one for each nucleotide) that are laser activated, and detection of the emitted wavelengths by a charge coupled device camera. Output/light intensity can be converted to electrical signal using appropriate software (e.g. GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA that might be present in limited amounts in a particular sample.

## *Obtaining Polypeptides*

Human asparagine-hydroxylase polypeptides can be obtained, for example, by purification from human cells, by expression of asparagine-hydroxylase polynucleotides, or by direct chemical synthesis.

## *Protein Purification*

Human asparagine-hydroxylase polypeptides can be purified from any cell that expresses the polypeptide, including host cells that have been transfected with asparagine-hydroxylase expression constructs. A purified asparagine-hydroxylase polypeptide is separated from other compounds that normally associate with the asparagine-hydroxylase polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis. A preparation of purified asparagine-hydroxylase polypeptides is at least 80% pure; preferably, the preparations are 90%, 95%, or 99% pure. Purity of the preparations can be assessed by any means known in the art, such as SDS-polyacrylamide gel electrophoresis.

*Expression of Polynucleotides*

To express a asparagine-hydroxylase polynucleotide, the polynucleotide can be inserted into an expression vector that contains the necessary elements for the transcription and translation of the inserted coding sequence. Methods that are well known to those skilled in the art can be used to construct expression vectors containing sequences encoding asparagine-hydroxylase polypeptides and appropriate transcriptional and translational control elements. These methods include *in vitro* recombinant DNA techniques, synthetic techniques, and *in vivo* genetic recombination. Such techniques are described, for example, in Sambrook *et al.* (1989) and in Ausubel et al., CURRENT PROTOCOLS IN MOLECULAR BIOLOGY, John Wiley & Sons, New York, N.Y., 1989.

A variety of expression vector/host systems can be utilized to contain and express sequences encoding a asparagine-hydroxylase polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those non-translated regions of the vectorenhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a asparagine-hydroxylase polypeptide, vectors based on SV40 or EBV can be used with an appropriate selectable marker.

*Bacterial and Yeast Expression Systems*

In bacterial systems, a number of expression vectors can be selected depending upon the use intended for the asparagine-hydroxylase polypeptide. For example, when a large quantity of a asparagine-hydroxylase polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the asparagine-hydroxylase polypeptide can be ligated into the vector in frame with sequences for the amino-terminal Met and the subsequent 7 residues of β-galactosidase so that a hybrid protein is produced. pIN vectors (Van Heeke & Schuster, J. Biol. Chem. 264, 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel *et al.* (1989) and Grant et al., Methods Enzymol. 153, 516-544, 1987.

*Plant and Insect Expression Systems*

If plant expression vectors are used, the expression of sequences encoding asparagine-hydroxylase polypeptides can be driven by any of a number of promoters. For example, viral promoters such as the 35S and 19S promoters of CaMV can be used alone or in combination with the omega leader sequence from TMV (Takamatsu, EMBO J. 6, 307-311, 1987). Alternatively, plant promoters such as the small subunit of RUBISCO or heat shock promoters can be used (Coruzzi et al., EMBO J. 3, 1671-1680, 1984; Broglie et al., Science 224, 838-843, 1984; Winter et al., Results Probl. Cell Differ. 17, 85-105, 1991). These constructs can be introduced into plant cells by direct DNA transformation or by pathogen-mediated transfection. Such techniques are described in a number of generally available reviews (e.g., Hobbs or Murray, in McGRAW HILL YEARBOOK OF SCIENCE AND TECHNOLOGY, McGraw Hill, New York, N.Y., pp. 191-196, 1992).

An insect system also can be used to express a asparagine-hydroxylase polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding asparagine-hydroxylase polypeptides can be cloned into a non-essential region of the virus, such as the polyhedrin gene, and placed under control of the polyhedrin promoter. Successful insertion of asparagine-hydroxylase polypeptides will render the polyhedrin gene inactive and produce recombinant virus lacking coat protein. The recombinant viruses can then be used to infect S. *frugiperda cells* or *Trichoplusia* larvae in which asparagine-hydroxylase polypeptides can be expressed (Engelhard et al., Proc. Nat. Acad. Sci. 91, 3224-3227, 1994).

*Mammalian Expression Systems*

A number of viral-based expression systems can be used to express asparagine-hydroxylase polypeptides in mammalian host cells. For example, if an adenovirus is used as an expression vector, sequences encoding asparagine-hydroxylase polypeptides can be ligated into an adenovirus transcription/translation complex comprising the late promoter and tripartite leader sequence. Insertion in a non-essential E1 or E3 region of the viral genome can be used to obtain a viable virus that is capable of expressing a asparagine-hydroxylase polypeptide in infected host cells (Logan & Shenk, Proc. Natl. Acad. Sci. 81, 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding asparagine-hydroxylase polypeptides. Such signals include the ATG initiation codon and adjacent sequences. In cases where sequences encoding a asparagine-hydroxylase polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf et al., Results Probl. Cell Differ. 20, 125-162, 1994).

*Host Cells*

A host cell strain can be chosen for its ability to regulate the expression of the inserted sequences or to process the expressed asparagine-hydroxylase polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells that have specific cellular machinery and characteristic mechanisms for post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express asparagine-hydroxylase polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced asparagine-hydroxylase sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines.

These include, but are not limited to, the herpes simplex virus thymidine kinase (Wigler et al., Cell 11, 223-32, 1977) and adenine phosphoribosyltransferase (Lowy et al., Cell 22, 817-23, 1980) genes which can be employed in *tk*$^-$ or *aprt*$^-$ cells, respectively. Also, antimetabolite, antibiotic, or herbicide resistance can be used as the basis for selection.

For example, *dhfr* confers resistance to methotrexate (Wigler et al., Proc. Natl. Acad. Sci. 77, 3567-70, 1980), *npt* confers resistance to the aminoglycosides, neomycin and G-418 (Colbere-Garapin et al., J. Mol. Biol. 150, 1-14, 1981), and *als* and *pat* confer resistance to chlorsulfuron and phosphinotricin acetyltransferase, respectively (Murray, 1992, *supra).* Additional selectable genes have been described. For example, *trpB* allows cells to utilize indole in place of tryptophan, or *hisD,* which allows cells to utilize histinol in place of histidine (Hartman & Mulligan, Proc. Natl. Acad. Sci. 85, 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes et al., Methods Mol. Biol. 55, 121-131, 1995).

*Detecting Expression*

Although the presence of marker gene expression suggests that the asparagine-hydroxylase polynucleotide is also present, its presence and expression may need to be confirmed. For example, if a sequence encoding a asparagine-hydroxylase polypeptide is inserted within a marker gene sequence, transformed cells containing sequences that encode a asparagine-hydroxylase polypeptide can be identified by the absence of marker gene function. Alternatively, a marker gene can be placed in tandem with a sequence encoding a asparagine-hydroxylase polypeptide under the control of a single promoter. Expression of the marker gene in response to induction or selection usually indicates expression of the asparagine-hydroxylase polynucleotide.

Alternatively, host cells which contain a asparagine-hydroxylase polynucleotide and which express a asparagine-hydroxylase polypeptide can be identified by a variety of procedures known to those of skill in the art. These procedures include, but are not limited to, DNA-DNA or DNA-RNA hybridizations and protein bioassay or immunoassay techniques that include membrane, solution, or chip-based technologies for the detection and/or quantification of nucleic acid or protein. For example, the presence of a polynucleotide sequence encoding a asparagine-hydroxylase polypeptide can be detected by DNA-DNA or DNA-RNA hybridization or amplification using probes or fragments or fragments of polynucleotides encoding a asparagine-hydroxylase polypeptide. Nucleic acid amplification-based assays involve the use of oligonucleotides selected from sequences encoding a asparagine-hydroxylase polypeptide to detect transformants that contain a asparagine-hydroxylase polynucleotide.

A variety of protocols for detecting and measuring the expression of a asparagine-hydroxylase polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a asparagine-hydroxylase polypeptide can be used, or a competitive binding assay can be employed. These and other assays are described in Hampton et al., SEROLOGICAL METHODS: A LABORATORY MANUAL, APS Press, St. Paul, Minn., 1990) and Maddox et al., J. Exp. Med. 158, 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding asparagine-hydroxylase polypeptides include oligolabeling, nick translation, end-labeling, or PCR amplification using a labeled nucleotide. Alternatively, sequences encoding a asparagine-hydroxylase polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a asparagine-hydroxylase polypeptide can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing polynucleotides which encode asparagine-hydroxylase polypeptides can be designed to contain signal sequences which direct secretion of soluble asparagine-hydroxylase polypeptides through a prokaryotic or eukaryotic cell membrane or which direct the membrane insertion of membrane-bound asparagine-hydroxylase polypeptide.

As discussed above, other constructions can be used to join a sequence encoding a asparagine-hydroxylase polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the asparagine-hydroxylase polypeptide also can be used to facilitate purification. One such expression vector provides for expression of a fusion protein containing a asparagine-hydroxylase polypeptide and 6 histidine residues preceding a thioredoxin or an enterokinase cleavage site. The histidine residues facilitate purification by IMAC (immobilized metal ion affinity chromatography, as described in Porath et al., Prot. Exp. Purif. 3, 263-281, 1992), while the enterokinase cleavage site provides a means for purifying the asparagine-hydroxylase polypeptide from the fusion protein. Vectors that contain fusion proteins are disclosed in Kroll et al., DNA Cell Biol. 12, 441-453, 1993.

*Chemical Synthesis*

Sequences encoding a asparagine-hydroxylase polypeptide can be synthesised, in whole or in part, using chemical methods well known in the art (see Caruthers et al., Nucl. Acids Res. Symp. Ser. 215-223, 1980; Horn et al. Nucl. Acids Res. Symp. Ser. 225-232, 1980). Alternatively, a asparagine-hydroxylase polypeptide itself can be produced using chemical methods to synthesise its amino acid sequence, such as by direct peptide synthesis using solid-phase techniques (Merrifield, J. Am. Chem. Soc. 85, 2149-2154, 1963; Roberge et al., Science 269, 202-204, 1995). Protein synthesis can be performed using manual techniques or by automation. Automated synthesis can be achieved, for example, using Applied Biosystems 431A Peptide Synthesizer (Perkin Elmer). Optionally, fragments of asparagine-hydroxylase polypeptides can be separately synthesised and combined using chemical methods to produce a full-length molecule.

The newly synthesised peptide can be substantially purified by preparative high performance liquid chromatography (e.g., Creighton, PROTEINS: STRUCTURES AND MOLECULAR PRINCIPLES, WH Freeman and Co., New York, N.Y., 1983). The composition of a synthetic asparagine-hydroxylase polypeptide can be confirmed by amino acid analysis or sequencing (e.g., the Edman degradation procedure; *see* Creighton, *supra).* Additionally, any portion of the amino acid sequence of the asparagine-hydroxylase polypeptide can be altered during direct synthesis and/or combined using chemical methods with sequences from other proteins to produce a variant polypeptide or a fusion protein.

*Production ofAltered Polypeptides*

As will be understood by those of skill in the art, it may be advantageous to produce asparagine-hydroxylase polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life that is longer than that of a transcript generated from the naturally occurring sequence.

The nucleotide sequences disclosed herein can be engineered using methods generally known in the art to alter asparagine-hydroxylase polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Antibodies*

Any type of antibody known in the art can be generated to bind specifically to an epitope of a asparagine-hydroxylase polypeptide. "Antibody" as used herein includes intact immunoglobulin molecules, as well as fragments thereof, such as Fab, $F(ab')_2$, and Fv, which are capable of binding an epitope of a asparagine-hydroxylase polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a asparagine-hydroxylase polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody that specifically binds to the immunogen.

Typically, an antibody which specifically binds to a asparagine-hydroxylase polypeptide provides a detection signal at least 5-, 10-, or 20-fold higher than a detection signal provided with other proteins when used in an immunochemical assay. Preferably, antibodies which specifically bind to asparagine-hydroxylase polypeptides do not detect other proteins in immunochemical assays and can immunoprecipitate a asparagine-hydroxylase polypeptide from solution.

Human asparagine-hydroxylase polypeptides can be used to immunize a mammal, such as a mouse, rat, rabbit, guinea pig, monkey, or human, to produce polyclonal antibodies. If desired, a asparagine-hydroxylase polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g. lysolecithin, pluronic polyols, polyanions, peptides, oil emulsions, keyhole limpet hemocyanin, and dinitrophenol). Among adjuvants used in humans, BCG *(bacilli Calmette-Guerin)* and *Corynebacterium parvum* are especially useful.

Monoclonal antibodies that specifically bind to a asparagine-hydroxylase polypeptide can be prepared using any technique which provides for the production of antibody molecules by continuous cell lines in culture. These techniques include, but are not limited to, the hybridoma technique, the human B-cell hybridoma technique, and the EBV-hybridoma technique (Kohler et al., Nature 256, 495-497, 1985; Kozbor et al., J Immunol. Methods 81, 31-42, 1985; Cote et al., Proc. Natl. Acad. Sci. 80, 2026-2030, 1983; Cole et al., Mol. Cell Biol. 62, 109-120, 1984).

In addition, techniques developed for the production of "chimeric antibodies," the splicing of mouse antibody genes to human antibody genes to obtain a molecule with appropriate antigen specificity and biological activity, can be used (Morrison et al., Proc. Natl. Acad. Sci. 81, 6851-6855, 1984; Neuberger et al., Nature 312, 604-608, 1984; Takeda et al., Nature 314, 452-454, 1985). Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimised by replacing residues which differ from those in the human sequences by site-directed mutagenesis of individual residues or by grating of entire complementarity determining regions. Alternatively, humanised antibodies can be produced using recombinant methods, as described in GB2188638B. Antibodies that specifically bind to a asparagine-hydroxylase polypeptide can contain antigen binding sites which are either partially or fully humanised, as disclosed in U.S. 5,565,332.

Alternatively, techniques described for the production of single chain antibodies can be adapted using methods known in the art to produce single chain antibodies that specifically bind to asparagine-hydroxylase polypeptides. Antibodies with related specificity, but of distinct idiotypic composition, can be generated by chain shuffling from random combinatorial immunoglobin libraries (Burton, Proc. Natl. Acad. Sci. 88, 11120-23, 1991).

Single-chain antibodies also can be constructed using a DNA amplification method, such as PCR, using hybridoma cDNA as a template (Thirion et al., 1996, Eur. J Cancer Prev. 5, 507-11). Single-chain antibodies can be mono- or bispecific, and can be bivalent or tetravalent. Construction of tetravalent, bispecific single-chain antibodies is taught, for example, in Coloma & Morrison, 1997, Nat. Biotechnol. 15, 159-63. Construction of bivalent, bispecific single-chain antibodies is taught in Mallender & Voss, 1994, J. Biol. Chem. 269, 199-206.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (Verhaar et al., 1995, Int. J. Cancer 61, 497-501; Nicholls et al., 1993, J Immunol. Meth. 165, 81-91).

Antibodies which specifically bind to asparagine-hydroxylase polypeptides also can be produced by inducing *in vivo* production in the lymphocyte population or by screening immunoglobulin libraries or panels of highly specific binding reagents as disclosed in the literature (Orlandi et al., Proc. Natl. Acad. Sci. 86,3833-3837, 1989; Winter et al., Nature 349, 293-299, 1991).

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which a asparagine-hydroxylase polypeptide is bound. The bound antibodies can then be eluted from the column using a buffer with a high salt concentration.

*Antisense Oligonucleotides*

Antisense oligonucleotides are nucleotide sequences that are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of asparagine-hydroxylase gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. *See* Brown, Meth. Mol. Biol. 20, 1-8, 1994; Sonveaux, Meth. Mol. Biol. 26, 1-72, 1994; Uhlmann et al., Chem. Rev. 90, 543-583, 1990.

Modifications of asparagine-hydroxylase gene expression can be obtained by designing antisense oligonucleotides that will form duplexes to the control, 5', or regulatory regions of the asparagine-hydroxylase gene. Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature (e.g., Gee et al., in Huber & Carr, MOLECULAR AND IMMUNOLOGIC APPROACHES, Futura Publishing Co., Mt. Kisco, N.Y., 1994). An antisense oligonucleotide also can be designed to block translation of mRNA by preventing the transcript from binding to ribosomes.

Precise complementarity is not required for successful complex formation between an antisense oligonucleotide and the complementary sequence of a asparagine-hydroxylase polynucleotide. Antisense oligonucleotides which comprise, for example, 2, 3, 4, or 5 or more stretches of contiguous nucleotides which are precisely complementary to a asparagine-hydroxylase polynucleotide, each separated by a stretch of contiguous nucleotides which are not complementary to adjacent asparagine-hydroxylase nucleotides, can provide sufficient targeting specificity for asparagine-hydroxylase mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular asparagine-hydroxylase polynucleotide sequence.

Antisense oligonucleotides can be modified without affecting their ability to hybridize to a asparagine-hydroxylase polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art. *See, e.g.,* Agrawal et al., Trends Biotechnol. 10, 152-158, 1992; Uhlmann et al., Chem. Rev. 90, 543-584, 1990; Uhlmann et al., Tetrahedron. Lett. 215, 3539-3542, 1987.

*Ribozymes*

Ribozymes are RNA molecules with catalytic activity. *See, e.g.,* Cech, Science 236, 1532-1539; 1987; Cech, Ann. Rev. Biochem. 59, 543-568; 1990, Cech, Curr. Opin. Struct. Biol. 2, 605-609; 1992, Couture & Stinchcomb, Trends Genet. 12, 510-515, 1996. Ribozymes can be used to inhibit gene function by cleaving an RNA sequence, as is known in the art (e.g., Haseloff et al., U.S. Patent 5,641,673). The mechanism of ribozyme action involves sequence-specific hybridisation of the ribozyme molecule to complementary target RNA, followed by endonucleolytic cleavage. Examples include engineered hammerhead motif ribozyme molecules that can specifically and efficiently catalyse endonucleolytic cleavage of specific nucleotide sequences.

The coding sequence of a asparagine-hydroxylase polynucleotide can be used to generate ribozymes that will specifically bind to mRNA transcribed from the asparagine-hydroxylase polynucleotide. Methods of designing and constructing ribozymes which can cleave other RNA molecules in trans in a highly sequence specific manner have been developed and described in the art *(see* Haseloff et al. Nature 334, 585-591, 1988). For example, the cleavage activity of ribozymes can be targeted to specific RNAs by engineering a discrete "hybridization" region into the ribozyme. The hybridisation region contains a sequence complementary to the target RNA and thus specifically hybridises with the target (see, for example, Gerlach et al., EP 321,201).

Specific ribozyme cleavage sites within a asparagine-hydroxylase RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate asparagine-hydroxylase RNA targets also can be evaluated by testing accessibility to hybridization with complementary oligonucleotides using ribonuclease protection assays. Longer complementary sequences can be used to increase the affinity of the hybridization sequence for the target. The hybridising and cleavage regions of the ribozyme can be integrally related such that upon hybridising to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease asparagine-hydroxylase expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff et al., U.S. Patent 5,641,673, ribozymes can be engineered so that ribozyme expression will occur in response to factors that induce expression of a target gene. Ribozymes also can be engineered to provide an additional level of regulation, so that destruction of mRNA occurs only when both a ribozyme and a target gene are induced in the cells.

*Differentially Expressed Genes*

Described herein are methods for the identification of genes whose products interact with human asparagine-hydroxylase. Such genes may represent genes that are differentially expressed in disorders including, but not limited to, anaemia, cancer, cardiovascular disorders, inflammatory diseases, fibrotic disorders, and CNS disorders. Further, such genes may represent genes that are differentially regulated in response to manipulations relevant to the progression or treatment of such diseases. Additionally, such genes may have a temporally regulated expression, increased or decreased at different stages of tissue or organism development. A differentially expressed gene may also have its expression regulated under control versus experimental conditions. In addition, the human asparagine-hydroxylase gene or gene product may itself be tested for differential expression.

The degree to which expression differs in a normal versus a diseased state need only be large enough to be visualised via standard characterisation techniques such as differential display techniques. Other such standard characterisation techniques by which expression differences may be visualised include but are not limited to, quantitative RT (reverse transcriptase), PCR, and Northern analysis.

*Identification ofDifferentially Expressed Genes*

To identify differentially expressed genes total RNA or, preferably, mRNA is isolated from tissues of interest. For example, RNA samples are obtained from tissues of experimental subjects and from corresponding tissues of control subjects. Any RNA isolation technique that does not select against the isolation of mRNA may be utilized for the purification of such RNA samples. See, for example, Ausubel et al., ed., CURRENT PROTOCOLS IN MOLECULAR BIOLOGY, John Wiley & Sons, Inc. New York, 1987-1993. Large numbers of tissue samples may readily be processed using techniques well known to those of skill in the art, such as, for example, the single-step RNA isolation process of Chomczynski, U.S. Patent 4,843,155.

Transcripts within the collected RNA samples that represent RNA produced by differentially expressed genes are identified by methods well known to those of skill in the art. They include, for example, differential screening (Tedder et al., Proc. Natl. Acad. Sci. U.S.A. 85, 208-12, 1988), subtractive hybridization (Hedrick et al., Nature 308, 149-53; Lee et al., Proc. Natl. Acad. Sci. U.S.A. 88, 2825, 1984), and, preferably, differential display (Liang & Pardee, Science 257, 967-71, 1992; U.S. Patent 5,262,311).

The differential expression information may itself suggest relevant methods for the treatment of disorders involving the human asparagine-hydroxylase. For example, treatment may include a regulation of expression of the differentially expressed genes and/or the gene encoding the human asparagine-hydroxylase. The differential expression information may indicate whether the expression or activity of the differentially expressed gene or gene product or the human asparagine-hydroxylase gene or gene product are up-regulated or down-regulated.

*Screening Methods*

The invention provides assays for screening test compounds that bind to or regulate the activity of a asparagine-hydroxylase polypeptide or a asparagine-hydroxylase polynucleotide. A test compound preferably binds to a asparagine-hydroxylase polypeptide or polynucleotide. More preferably, a test compound decreases or increases asparagine-hydroxylase activity by at least about 10, preferably about 50, more preferably about 75, 90, or 100% relative to the absence of the test compound.

*Test Compounds*

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesised by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des. 12,* 145, 1997.

Methods for the synthesis of molecular libraries are well known in the art *(see,* for example, DeWitt et al., Proc. Natl. Acad. Sci. U.S.A. 90, 6909, 1993; Erb et al. Proc. Natl. Acad. Sci. U.S.A. 91, 11422, 1994; Zuckermann et al., J. Med. Chem. 37, 2678, 1994; Cho et al., Science 261, 1303, 1993; Carell et al., Angew. Chem. Int. Ed. Engl. 33, 2059, 1994; Carell et al., Angew. Chem. Int. Ed. Engl. 33, 2061; Gallop et al., J. Med. Chem. 37, 1233, 1994). Libraries of compounds can be presented in solution *(see, e.g.,* Houghten, BioTechniques 13, 412-421, 1992), or on beads (Lam, Nature 354, 82-84, 1991), chips (Fodor, Nature 364, 555-556, 1993), bacteria or spores (Ladner, U.S. Patent 5,223, 409), plasmids (Cull et al., Proc. Natl. Acad. Sci. U.S.A. 89, 1865-1869, 1992), or phage (Scott & Smith, Science 249, 386-390, 1990; Devlin, Science 249, 404-406, 1990); Cwirla et al., Proc. Natl. Acad. Sci. 97, 6378-6382, 1990; Felici, J. Mol. Biol. 222, 301-310, 1991; and Ladner, U.S. Patent 5,223,409).

*High Throughput Screening*

Test compounds can be screened for the ability to bind to asparagine-hydroxylase polypeptides or polynucleotides or to affect asparagine-hydroxylase activity or asparagine-hydroxylase gene expression using high throughput screening. Using high throughput screening, many discrete compounds can be tested in parallel so that large numbers of test compounds can be quickly screened. The most widely established techniques utilise 96-well, 384-well or 1536-well microtiter plates. The wells of the microtiter plates typically require assay volumes that range from 5 to 500 μl. In addition to the plates, many instruments, materials, pipettors, robotics, plate washers, and plate readers are commercially available to fit the microwell formats.

Alternatively, "free format assays," or assays that have no physical barrier between samples, can be used. For example, an assay using pigment cells (melanocytes) in a simple homogeneous assay for combinatorial peptide libraries is described by Jayawickreme et al., Proc. Natl. Acad. Sci. U.S.A. 19, 1614-18 (1994). The cells are placed under agarose in petri dishes, then beads that carry combinatorial compounds are placed on the surface of the agarose. The combinatorial compounds are partially released the compounds from the beads. Active compounds can be visualised as dark pigment areas because, as the compounds diffuse locally into the gel matrix, the active compounds cause the cells to change colours.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a colour change throughout the gel. Thereafter, beads carrying combinatorial compounds via a photolinker were placed inside the gel and the compounds were partially released by UV-light. Compounds that inhibited the enzyme were observed as local zones of inhibition having less colour change.

Yet another example is described by Salmon et al., Molecular Diversity 2, 57-63 (1996). In this example, combinatorial libraries were screened for compounds that had cytotoxic effects on cancer cells growing in agar.

Another high throughput screening method is described in Beutel et al., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule that binds to and occupies, for example, the active site of the asparagine-hydroxylase polypeptide, such that normal biological activity is prevented. Examples of such small molecules include, but are not limited to, small peptides or peptide-like molecules.

In binding assays, either the test compound or the asparagine-hydroxylase polypeptide can comprise a detectable label, such as a fluorescent, radioisotopic, chemiluminescent, or enzymatic label, such as horseradish peroxidase, alkaline phosphatase, or luciferase. Detection of a test compound that is bound to the asparagine-hydroxylase polypeptide can then be accomplished, for example, by direct counting of radioemmission, by scintillation counting, or by determining conversion of an appropriate substrate to a detectable product.

Alternatively, binding of a test compound to a asparagine-hydroxylase polypeptide can be determined without labeling either of the interactants. For example, a microphysiometer can be used to detect binding of a test compound with a asparagine-hydroxylase polypeptide. A microphysiometer (e.g., Cytosensor™) is an analytical instrument that measures the rate at which a cell acidifies its environment using a light-addressable potentiometric sensor (LAPS). Changes in this acidification rate can be used as an indicator of the interaction between a test compound and a asparagine-hydroxylase polypeptide (McConnell et al., Science 257, 1906-1912, 1992).

Determining the ability of a test compound to bind to a asparagine-hydroxylase polypeptide also can be accomplished using a technology such as real-time Bimolecular Interaction Analysis (BIA) (Sjolander & Urbaniczky, Anal. Chem. 63, 2338-2345, 1991, and Szabo et al., Curr. Opin. Struct. Biol. 5, 699-705, 1995). BIA is a technology for studying biospecific interactions in real time, without labeling any of the interactants (e.g., BIAcore™). Changes in the optical phenomenon surface plasmon resonance (SPR) can be used as an indication of real-time reactions between biological molecules.

In yet another aspect of the invention, a asparagine-hydroxylase polypeptide can be used as a "bait protein" in a two-hybrid assay or three-hybrid assay (see, *e.g.,* U.S. Patent 5,283,317; Zervos et al., Cell 72, 223-232, 1993; Madura et al., J. Biol. Chem. 268, 12046-12054, 1993; Bartel et al., BioTechniques 14, 920-924, 1993; Iwabuchi et al., Oncogene 8, 1693-1696, 1993; and Brent W094/10300), to identify other proteins which bind to or interact with the asparagine-hydroxylase polypeptide and regulate its activity.

The two-hybrid system is based on the modular nature of most transcription factors, which consist of separable DNA-binding and activation domains. Briefly, the assay utilizes two different DNA constructs. For example, in one construct, polynucleotide encoding a asparagine-hydroxylase polypeptide can be fused to a polynucleotide encoding the DNA binding domain of a known transcription factor (e.g., GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein that interacts with the asparagine-hydroxylase polypeptide.

It may be desirable to immobilise either the asparagine-hydroxylase polypeptide (or polynucleotide) or the test compound to facilitate separation of bound from unbound forms of one or both of the interactants, as well as to accommodate automation of the assay. Thus, either the asparagine-hydroxylase polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the enzyme polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a asparagine-hydroxylase polypeptide (or polynucleotide) can be accomplished in any vessel suitable for containing the reactants. Examples of such vessels include microtiter plates, test tubes, and microcentrifuge tubes.

In one embodiment, the asparagine-hydroxylase polypeptide is a fusion protein comprising a domain that allows the asparagine-hydroxylase polypeptide to be bound to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed asparagine-hydroxylase polypeptide; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilising proteins or polynucleotides on a solid support also can be used in the screening assays of the invention. For example, either a asparagine-hydroxylase polypeptide (or polynucleotide) or a test compound can be immobilised utilising conjugation of biotin and streptavidin. Biotinylated asparagine-hydroxylase polypeptides (or polynucleotides) or test compounds can be prepared from biotin-NHS(N-hydroxysuccinimide) using techniques well known in the art *(e.g.,* biotinylation kit, Pierce Chemicals, Rockford, Ill.) and immobilized in the wells of streptavidin-coated 96 well plates (Pierce Chemical). Alternatively, antibodies which specifically bind to a asparagine-hydroxylase polypeptide, polynucleotide, or a test compound, but which do not interfere with a desired binding site, such as the active site of the asparagine-hydroxylase polypeptide, can be derivatized to the wells of the plate. Unbound target or protein can be trapped in the wells by antibody conjugation.

Methods for detecting such complexes, in addition to those described above for the GST-immobilised complexes, include immunodetection of complexes using antibodies which specifically bind to the asparagine-hydroxylase polypeptide or test compound, enzyme-linked assays which rely on detecting an activity of the asparagine-hydroxylase polypeptide, and SDS gel electrophoresis under non-reducing conditions.

Screening for test compounds which bind to a asparagine-hydroxylase polypeptide or polynucleotide also can be carried out in an intact cell. Any cell which comprises a asparagine-hydroxylase polypeptide or polynucleotide can be used in a cell-based assay system. A asparagine-hydroxylase polynucleotide can be naturally occurring in the cell or can be introduced using techniques such as those described above. Binding of the test compound to a asparagine-hydroxylase polypeptide or polynucleotide is determined as described above.

*Enzyme Assays*

Test compounds can be tested for the ability to regulate (increase or decrease) the hydroxylase activity of a human asparagine hydroxylase polypeptide or a polypeptide exhibiting the biological activity of an asparagine hydroxylase. Asparagine hydroxylase activity can be measured, for example, as described in Kivirikko, K. I., and Myllylä, T. (1982) Methods Enzymol. 82, 245-304; Cuncliffe, C. J., Franklin, T. J., and Gaskell, R. M. (1986) Biochem. J. 240, 617-619; Kaule, G., and Günzler, V. (1990) Anal. Biochem. 184, 291-297.

Enzyme assays can be carried out after contacting either a purified asparagine hydroxylase polypeptide, a cell membrane preparation, or an intact cell with a test compound. A test compound that decreases a asparagine hydroxylase activity of a asparagine hydroxylase polypeptide by at least about 10, preferably about 50, more preferably about 75, 90, or 100% is identified as a potential therapeutic agent for decreasing asparagine hydroxylase activity. A test compound which increases a asparagine hydroxylase activity of a human asparagine hydroxylase polypeptide by at least about 10, preferably about 50, more preferably about 75, 90, or 100% is identified as a potential therapeutic agent for increasing human asparagine hydroxylase activity.

*Gene Expression*

In another embodiment, test compounds that increase or decrease asparagine-hydroxylase gene expression are identified. A asparagine-hydroxylase polynucleotide is contacted with a test compound, and the expression of an RNA or polypeptide product of the asparagine-hydroxylase polynucleotide is determined. The level of expression of appropriate mRNA or polypeptide in the presence of the test compound is compared to the level of expression of mRNA or polypeptide in the absence of the test compound. The test compound can then be identified as a regulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of asparagine-hydroxylase mRNA or polypeptide expression in the cells can be determined by methods well known in the art for detecting mRNA or polypeptide. Either qualitative or quantitative methods can be used. The presence of polypeptide products of a asparagine-hydroxylase polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labeled amino acids into a asparagine-hydroxylase polypeptide.

Such screening can be carried out either in a cell-free assay system or in an intact cell. Any cell that expresses a asparagine-hydroxylase polynucleotide can be used in a cell-based assay system. The asparagine-hydroxylase polynucleotide can be naturally occurring in the cell or can be introduced using techniques such as those described above. Either a primary culture or an established cell line, such as CHO or human embryonic kidney 293 cells, can be used.

*Pharmaceutical Compositions*

The invention also provides pharmaceutical compositions that can be administered to a patient to achieve a therapeutic effect. Pharmaceutical compositions of the invention can comprise, for example, a asparagine-hydroxylase polypeptide, asparagine-hydroxylase polynucleotide, ribozymes or antisense oligonucleotides, antibodies which specifically bind to a asparagine-hydroxylase polypeptide, or mimetics, activators, or inhibitors of a asparagine-hydroxylase polypeptide activity. The compositions can be administered alone or in combination with at least one other agent, such as stabilising compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically-acceptable carriers comprising excipients and auxiliaries that facilitate processing of the active compounds into preparations which can be used pharmaceutically. Pharmaceutical compositions of the invention can be administered by any number of routes including, but not limited to, oral, intravenous, intramuscular, intra-arterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterise the quantity of active compound, i.e., dosage.

Pharmaceutical preparations that can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilisers. In soft capsules, the active compounds can be dissolved or suspended in suitable liquids, such as fatty oils, liquid, or liquid polyethylene glycol with or without stabilisers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances that increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents that increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known in the art, e.g., by means of conventional mixing, dissolving, granulating, dragee-making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilised powder which can contain any or all of the following: 1-50 mM histidine, 0.1%-2% sucrose, and 2-7% mannitol, at a pH range of 4.5 to 5.5, that is combined with buffer prior to use.

Further details on techniques for formulation and administration can be found in the latest edition of REMINGTON'S PHARMACEUTICAL SCIENCES (Maack Publishing Co., Easton, Pa.). After pharmaceutical compositions have been prepared, they can be placed in an appropriate container and labeled for treatment of an indicated condition. Such labeling would include amount, frequency, and method of administration.

*Therapeutic Indications and Methods*

The novel human asparagine-hydroxylases ASNH-1, ASNH-2, and ASNH-3 can be regulated to treat cardiovascular disorders, anaemia, cancer, CNS disorders, inflammatory diseases, and fibrotic disorders. ASNH-1, ASNH-2, and ASNH-3 show a widespread tissue distribution which corresponds with a central function in oxygen sensing and/or posttranslational modification of still unidentified proteins. As asparagine-hydroxylases specific for hypoxia inducible transcription factors (HIFs), ASNH-1-3 play a central role in the regulation of those genes that are transcriptionally regulated in response to changes of tissue oxygenation. Among those HIF regulated genes, for example, vascular endothelial growth factor (VEGF) is of central importance for the de-novo formation of blood vessels (angiogenesis).

LIT and erythropoietin (Epo) is the key regulator of the formation of red blood cells (erythropoiesis) in the bone marrow.

*Cancer*

Cancer is a disease fundamentally caused by oncogenic cellular transformation. There are several hallmarks of transformed cells that distinguish them from their normal counterparts and underlie the pathophysiology of cancer. These include uncontrolled cellular proliferation, unresponsiveness to normal death-inducing signals (immortalization), increased cellular motility and invasiveness, increased ability to recruit blood supply through induction of new blood vessel formation (angiogenesis), genetic instability, and dysregulated gene expression. Various combinations of these aberrant physiologies, along with the acquisition of drug-resistance frequently lead to an intractable disease state in which organ failure and patient death ultimately ensue.

Most standard cancer therapies target cellular proliferation and rely on the differential proliferative capacities between transformed and normal cells for their efficacy. This approach is hindered by the facts that several important normal cell types are also highly proliferative and that cancer cells frequently become resistant to these agents. Thus, the therapeutic indices for traditional anti-cancer therapies rarely exceed 2.0.

The advent of genomics-driven molecular target identification has opened up the possibility of identifying new cancer-specific targets for therapeutic intervention that will provide safer, more effective treatments for cancer patients. Thus, newly discovered tumor-associated genes and their products can be tested for their role(s) in disease and used as tools to discover and develop innovative therapies. Genes playing important roles in any of the physiological processes outlined above can be characterized as cancer targets.

Genes or gene fragments identified through genomics can readily be expressed in one or more heterologous expression systems to produce functional recombinant proteins. These proteins are characterized *in vitro* for their biochemical properties and then used as tools in high-throughput molecular screening programs to identify chemical modulators of their biochemical activities. Agonists and/or antagonists of target protein activity can be identified in this manner and subsequently tested in cellular and *in vivo* disease models for anti-cancer activity. Optimization of lead compounds with iterative testing in biological models and detailed pharmacokinetic and toxicological analyses form the basis for drug development and subsequent testing in humans.

There are more than 100 different varieties of cancer, which can be divided into six major categories depending on their cell type or site of origin: Carcinomas, the most common type of cancer, are epithelial in origin; sarcomas originate in connective tissues; lymphomas are cancers of the lymph system; leukemias involve blood-forming tissues and blood cells; brain tumors are cancers that originate in brain; skin cancers, including melanomas, originate in skin.

The four most common cancers are: Breast cancer, prostrate cancer, lung cancer and colon cancer.

Breast cancer, a common cancer in women, is a disease in which malignant cells are found in the tissues of the breast. Roughly 180,000 women are diagnosed with this disease each year, of which 44,000 will die. Each breast has 15 to 20 sections called lobes, which have many smaller sections called lobules. The lobes and lobules are connected by thin tubes called ducts. The most common type of breast cancer is ductal cancer. It is found in the cells of the ducts. Cancer that begins in the lobes or lobules is called lobular cancer. Lobular cancer is more often found in both breasts than other types of breast cancer. Hereditary breast cancer makes up approximately 5% to 10% of all breast cancer cases.

Prostrate cancer is the most common cancer in aging men. It is estimated that 40% of men over age of 50 have microscopic areas of cancer in their prostate gland, however only 8% of men will develop clinically significant disease and only 3% will die of this disease. The prostrate is about the size of a walnut surrounding the upper part of the urethra, the tube that carries urine and semen out of the penis. It produces some of the seminal fluid which protects and nourishes the sperms. Prostrate cancer could spread to other parts of the body such as bone and lungs.

Lung cancer is the second most common malignancy affecting both sexes. It is considered the most rapidly increasing cause of death from cancer. Smoking is considered to be the prime risk factor for lung cancer. There are two basic kinds of lung cancer: Small cell or oat cell, that occurs in one-third of all patients with lung cancer and Non-small cell in two-thirds of patients.

Colorectal cancer is the third most common cancer in men and women. The specific cause of Colorectal cancer is unknown, however, environmental, genetic, familial factors and preexisting Ulcerative Colitis have been linked to the development of this cancer. Most colorectal cancers develop slowly, beginning as small benign colorectal adenomas that progress over several decades to larger and more dysplastic lesions that eventually become malignant.

*Cardiovascular disorders*

Cardiovascular diseases include the following disorders of the heart and the vascular system: congestive heart failure, myocardial infarction, ischemic diseases of the heart, all kinds of atrial and ventricular arrhythmias, hypertensive vascular diseases, and peripheral vascular diseases.

Heart failure is defined as a pathophysiologic state in which an abnormality of cardiac function is responsible for the failure of the heart to pump blood at a rate commensurate with the requirement of the metabolising tissue. It includes all forms of pumping failure, such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included, as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina, and asymptomatic ischemia.

Arrhythmias include all forms of atrial and ventricular tachyarrhythmias (atrial tachycardia, atrial flutter, atrial fibrillation, atrio-ventricular reentrant tachycardia, preexcitation syndrome, ventricular tachycardia, ventricular flutter, and ventricular fibrillation), as well as bradycardic forms of arrhythmias.

Vascular diseases include primary as well as all kinds of secondary arterial hypertension (renal, endocrine, neurogenic, others). The disclosed genes and their products may be used as drug targets for the treatment of hypertension as well as for the prevention of all complications.

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon, and venous disorders.

*Inflammatory diseases*

Inflammatory diseases are characterised by tissue alteration and/or destruction by cells and/or products of the body's immune defence system, either in response to exogenous agents, such as viral or bacterial pathogens or chemical agents, and/or in response to normal or altered structures (e.g., auto-immune diseases). Inflammatory tissue alterations include delivery of plasma water as consequence of disturbed blood vessel permeability, deposition of immune defense cells, deposition of collagen with tissue induration and scar formation, destruction of tissue, and *de novo* formation of blood vessels. Inflammatory diseases include acute and chronic alterations of the joints, such as rheumatoid arthritis, of the skin, such as psoriasis of the heart and other inner organs, such as lupus erythematosus, and forms of myocarditis. The disclosed genes and their products may be used as drug targets for the treatment of inflammatory diseases.

*Fibrotic disorders*

Fibrotic disorders originate either as a secondary response to tissue alterations, such as toxic or inflammatory destruction of the liver, or as primary lesions without discernible aetiology. They are characterised by overproduction and deposit of collagen into the interstitium of the diseased organs, resulting in severely impaired organ function. Fibrotic disorders include fibrotic alterations of the skin, the liver, the lung, and the heart.

*Anaemia*

Anaemias are characterised by a lack of oxygen-transporting red blood cells. This leads to an impaired tissue oxygen supply. The lack of red blood cells can be the result of bleeding, of increased destruction of red blood cells (e.g., due to toxic agents), decreased red blood cell stability, or to decreased *de-novo* formation of red blood cells in the bone marrow. Impaired *de-novo* formation can be the result of exposure to toxic agents (e.g., cancer chemotherapeutic agents), infiltration of the bone marrow by cancer cells, or a lack of erythropoietin, which is an indispensable growth factor for red blood cell formation. Erythropoietin is mainly, but not exclusively, secreted from the kidney. Therefore, the latter kind of anemia is mainly, but not exclusively, observed in patients with alterations of the kidneys.

*CNS disorders*

Central and peripheral nervous system disorders also can be treated, such as primary and secondary disorders after brain injury, disorders of mood, anxiety disorders, disorders of thought and volition, disorders of sleep and wakefulness, diseases of the motor unit, such as neurogenic and myopathic disorders, neurodegenerative disorders such as Alzheimer's and Parkinson's disease, and processes of peripheral and chronic pain.

Pain that is associated with CNS disorders also can be treated by regulating the activity of human asparagine-hydroxylase. Pain which can be treated includes that associated with central nervous system disorders, such as multiple sclerosis, spinal cord injury, sciatica, failed back surgery syndrome, traumatic brain injury, epilepsy, Parkinson's disease, post-stroke, and vascular lesions in the brain and spinal cord (e.g., infarct, haemorrhage, vascular malformation). Non-central neuropathic pain includes that associated with post mastectomy pain, reflex sympathetic dystrophy (RSD), trigeminal neuralgiaradioculopathy, post-surgical pain, HIV/AIDS related pain, cancer pain, metabolic neuropathies (e.g., diabetic neuropathy, vasculitic neuropathy secondary to connective tissue disease), paraneoplastic polyneuropathy associated, for example, with carcinoma of lung, or leukaemia, or lymphoma, or carcinoma of prostate, colon or stomach, trigeminal neuralgia, cranial neuralgias, and post-herpetic neuralgia. Pain associated with cancer and cancer treatment also can be treated, as can headache pain (for example, migraine with aura, migraine without aura, and other migraine disorders), episodic and chronic tension-type headache, tension-type like headache, cluster headache, and chronic paroxysmal hemicrania.

Furthermore, the disclosed genes and their products can be used as drug targets for the prevention and the treatment of all CNS disorders that are due to alterations of brain blood vessels and/or due to ischemic and/or hypoxic alterations of the CNS.

This invention further pertains to the use of novel agents identified by the screening assays described above. Accordingly, it is within the scope of this invention to use a test compound identified as described herein in an appropriate animal model. For example, an agent identified as described herein (e.g., a regulating agent, an antisense nucleic acid molecule, a specific antibody, ribozyme, or a asparagine-hydroxylase polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

A reagent which affects asparagine-hydroxylase activity can be administered to a human cell, either *in vitro* or *in vivo,* to reduce asparagine-hydroxylase activity. The reagent preferably binds to an expression product of a human asparagine-hydroxylase gene. If the expression product is a protein, the reagent is preferably an antibody. For treatment of human cells *ex vivo,* an antibody can be added to a preparation of stem cells that have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5 $\mu$g of DNA per 16 nmole of liposome delivered to about $10^6$ cells, more preferably about 1.0 $\mu$g of DNA per 16 nmole of liposome delivered to about $10^6$ cells, and even more preferably about 2.0 $\mu$g of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes for use in the present invention include those liposomes standardly used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a particular cell type, such as a cell-specific ligand exposed on the outer surface of the liposome.

Complexing a liposome with a reagent such as an antisense oligonucleotide or ribozyme can be achieved using methods that are standard in the art (see, for example, U.S. Patent 5,705,151). Preferably, from about 0.1 $\mu$g to about 10 $\mu$g of polynucleotide is combined with about 8 nmol of liposomes, more preferably from about 0.5 $\mu$g to about 5 $\mu$g of polynucleotides are combined with about 8 nmol liposomes, and even more preferably about 1.0 $\mu$g of polynucleotides is combined with about 8 nmol liposomes.

In another embodiment, antibodies can be delivered to specific tissues *in vivo* using receptor-mediated targeted delivery. Receptor-mediated DNA delivery techniques are taught in, for example, Findeis et al. Trends in Biotechnol. 11, 202-05 (1993); Chiou et al., GENE THERAPEUTICS: METHODS AND APPLICATIONS OF DIRECT GENE TRANSFER (J.A. Wolff, ed.) (1994); Wu & Wu, J. Biol. Chem. 263, 621-24 (1988); Wu et al., J Biol. Chem. 269, 542-46 (1994) ; Zenke et al., Proc. Natl. Acad. Sci. U.S.A. 87, 3655-59 (1990); Wu et al., J. Biol. Chem. 266, 338-42 (1991).

*Determination of a Therapeutically Effective Dose*

The determination of a therapeutically effective dose is well within the capability of those skilled in the art. A therapeutically effective dose refers to that amount of active ingredient which increases or decreases asparagine-hydroxylase activity relative to the asparagine-hydroxylase activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$.

Pharmaceutical compositions that exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors that can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

Effective *in vivo* dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective *in vivo* dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides that express antisense oligonucleotides or ribozymes can be introduced into cells by a variety of methods, as described above.

Preferably, a reagent reduces expression of a asparagine-hydroxylase gene or the activity of a asparagine-hydroxylase polypeptide by at least about 10, preferably about 50, more preferably about 75, 90, or 100% relative to the absence of the reagent. The effectiveness of the mechanism chosen to decrease the level of expression of a asparagine-hydroxylase gene or the activity of a asparagine-hydroxylase polypeptide can be assessed using methods well known in the art, such as hybridisation of nucleotide probes to asparagine-hydroxylase-specific mRNA, quantitative RT-PCR, immunologic detection of a asparagine-hydroxylase polypeptide, or measurement of asparagine-hydroxylase activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, mammals such as dogs, cats, cows, horses, rabbits, monkeys, and most preferably, humans.

*Diagnostic Methods*

Human asparagine-hydroxylase also can be used in diagnostic assays for detecting diseases and abnormalities or susceptibility to diseases and abnormalities related to the presence of mutations in the nucleic acid sequences that encode the enzyme. For example, differences can be determined between the cDNA or genomic sequence encoding asparagine-hydroxylase in individuals afflicted with a disease and in normal individuals. If a mutation is observed in some or all of the afflicted individuals but not in normal individuals, then the mutation is likely to be the causative agent of the disease.

Sequence differences between a reference gene and a gene having mutations can be revealed by the direct DNA sequencing method. In addition, cloned DNA segments can be employed as probes to detect specific DNA segments. The sensitivity of this method is greatly enhanced when combined with PCR. For example, a sequencing primer can be used with a double-stranded PCR product or a single-stranded template molecule generated by a modified PCR. The sequence determination is performed by conventional procedures using radio-labelled nucleotides or by automatic sequencing procedures using fluorescent tags.

Genetic testing based on DNA sequence differences can be carried out by detection of alteration in electrophoretic mobility of DNA fragments in gels with or without denaturing agents. Small sequence deletions and insertions can be visualized, for example, by high resolution gel electrophoresis. DNA fragments of different sequences can be distinguished on denaturing formamide gradient gels in which the mobilities of different DNA fragments are retarded in the gel at different positions according to their specific melting or partial melting temperatures *(see, e.g.,* Myers et al., Science 230, 1242, 1985). Sequence changes at specific locations can also be revealed by nuclease protection assays, such as RNase and S 1 protection or the chemical cleavage method (e.g., Cotton et al., Proc. Natl. Acad. Sci. USA 85, 4397-4401, 1985). Thus, the detection of a specific DNA sequence can be performed by methods such as hybridisation, RNase protection, chemical cleavage, direct DNA sequencing or the use of restriction enzymes and Southern blotting of genomic DNA. In addition to direct methods such as gel-electrophoresis and DNA sequencing, mutations can also be detected by *in situ* analysis.

Altered levels of asparagine-hydroxylase also can be detected in various tissues. Assays used to detect levels of the receptor polypeptides in a body sample, such as blood or a tissue biopsy, derived from a host are well known to those of skill in the art and include radioimmunoassays, competitive binding assays, Western blot analysis, and ELISA assays.

All patents and patent applications cited in this disclosure are expressly incorporated herein by reference. The above disclosure generally describes the present invention. A more complete understanding can be obtained by reference to the following specific examples, which are provided for purposes of illustration only and are not intended to limit the scope of the invention.

## EXAMPLE 1

*Expression of recombinant human asparagine-hydroxylase*

The *Pichia pastoris* expression vector pPICZB (Invitrogen, San Diego, CA) is used to produce large quantities of recombinant human asparagine-hydroxylase polypeptides in yeast. The asparagine-hydroxylase-encoding DNA sequence is derived from SEQ ID NO: 1, 3 or 5. Before insertion into vector pPICZB, the DNA sequence is modified by well known methods in such a way that it contains at its 5'-end an initiation codon and at its 3'-end an enterokinase cleavage site, a His6 reporter tag and a termination codon. Moreover, at both termini recognition sequences for restriction endonucleases are added and after digestion of the multiple cloning site of pPICZ B with the corresponding restriction enzymes the modified DNA sequence is ligated into pPICZB. This expression vector is designed for inducible expression in *Pichia pastoris,* driven by a yeast promoter. The resulting pPICZ/md-His6 vector is used to transform the yeast.

The yeast is cultivated under usual conditions in 5 liter shake flasks and the recombinantly produced protein isolated from the culture by affinity chromatography (Ni-NTA-Resin) in the presence of 8 M urea. The bound polypeptide is eluted with buffer, pH 3.5, and neutralised. Separation of the polypeptide from the His6 reporter tag is accomplished by site-specific proteolysis using enterokinase (Invitrogen, San Diego, CA) according to manufacturer's instructions. Purified human asparagine-hydroxylase polypeptide is obtained.

## EXAMPLE 2

*Identification of test compounds that bind to asparagine-hydroxylase polypeptides*

Purified asparagine-hydroxylase polypeptides comprising a glutathione-S-transferase protein and absorbed onto glutathione-derivatized wells of 96-well microtiter plates are contacted with test compounds from a small molecule library at pH 7.0 in a physiological buffer solution. Human asparagine-hydroxylase polypeptides comprise the amino acid sequence shown in SEQ ID NOs: 2, 4 and 6. The test compounds comprise a fluorescent tag. The samples are incubated for 5 minutes to one hour. Control samples are incubated in the absence of a test compound.

The buffer solution containing the test compounds is washed from the wells. Binding of a test compound to a asparagine-hydroxylase polypeptide is detected by fluorescence measurements of the contents of the wells. A test compound that increases the fluorescence in a well by at least 15% relative to fluorescence of a well in which a test compound is not incubated is identified as a compound which binds to a asparagine-hydroxylase polypeptide.

## EXAMPLE 3

*Identification of a test compound which decreases asparagine-hydroxylase gene expression*

A test compound is administered to a culture of human cells transfected with a asparagine-hydroxylase expression construct and incubated at 37°C for 10 to 45 minutes. A culture of the same type of cells that have not been transfected is incubated for the same time without the test compound to provide a negative control.

RNA is isolated from the two cultures as described in Chirgwin et al., Biochem. 18, 5294-99, 1979). Northern blots are prepared using 20 to 30 μg total RNA and hybridized with a $^{32}P$-labeled asparagine-hydroxylase-specific probe at 65°C in Express-hyb (CLONTECH). The probe comprises at least 11 contiguous nucleotides selected from the complement of SEQ ID NO: 1, 3 or 5. A test compound that decreases the asparagine-hydroxylase-specific signal relative to the signal obtained in the absence of the test compound is identified as an inhibitor of asparagine-hydroxylase gene expression.

## EXAMPLE 4

*Identification of a test compound which decreases or increases asparagine hydroxylase activity*

A test compound is administered to a mixture of purified asparagine hydroxylase and an appropriate reaction buffer and incubated at 37°C for 10 to 45 minutes. A mixture of the same type but without test compound is used as a control. The asparagine hydroxylase activity is measured using a method of Kivirikko, K. I., Myllylä, T. (1982) Methods Enzymol. 82, 245-304; Cuncliffe, C. J., Franklin, T. J., Gaskell, R. M. (1986) Biochem. J. 240, 617-619, Kaule, G., and Günzler, V. (1990) Anal. Biochem. 184, 291-297.

A test compound which decreases the asparagine hydroxylase activity of the asparagine hydroxylase relative to the asparagine hydroxylase activity in the absence of the test compound is identified as an inhibitor of asparagine hydroxylase activity. A test compound which increases the asparagine hydroxylase activity of the asparagine hydroxylase relative to the asparagine hydroxylase activity in the absence of the test compound is identified as an activator of asparagine hydroxylase activity.

## EXAMPLE 5

***Detection of HIF- asparagine-hydroxylase activity***

HIF- asparagine hydroxylase activity of ASNH-3 was examined in a cellular co-transfection assay in HEK/293 cells. Cells were seeded at a density of 2 x $10^4$ cells per well in 96-well tissue culture plates (Greiner, Germany) and grown in DMEM F12 tissue culture medium (Gibco) supplemented with 10% fetal calf serum (PAA) and antibiotics for 24 hours at 37°C in a humidified tissue culture incubator (Heraeus, Germany) in a 5% $CO_2$ atmosphere. Plasmid DNA was introduced into the HEK/293 cells by use of Lipofectamin reagent (Gibco) according to the manufacturer's instructions.

The following expression plasmids were used in transfection experiments. pCDNA3 cloning vectors were obtained from Invitrogen, pFA-CMV and pFR-luc were from Stratagene:

1. pFA HIF-lalpha TADC containing the coding sequence for amino acids 736 to 836 of mouse hypoxia inducible factor-1 alpha (Gene bank accession number: NM_010431) carboxyterminally fused to the DNA binding domain of yeast GAL4 transcription factor (Gene bank accession number: P04386) contained in the pFA-CMV cloning vector.

2. pFA HIF-1 alpha N813A pCDNA3 containing the coding sequence of mouse hypoxia inducible factor-1 alpha in which the HIF asparagine hydroxylase sensitive asparagine residue at position 813 was replaced with alanine by site-directed mutagenesis using QuickChange™ XL Site-Directed Mutagenesis Kit, Stratagene. N813 is the mouse orthologue of human N803.

3. pFA HIF-2alpha TADC containing the coding sequence for amino acids 774 to 874 of mouse hypoxia inducible factor-2 alpha (Gene bank accession number: AF045160) carboxyterminally fused to the DNA binding domain of yeast GAL4 transcription factor (Gene bank accession number: P04386) contained in the pFA-CMV cloning vector.

4. pFA HIF-2 alpha N851A pCDNA3 containing the coding sequence of mouse hypoxia inducible factor-2 alpha in which the HIF asparagine hydroxylase sensitive asparagine residue at position 851 was replaced with alanine by site-directed mutagenesis using QuickChange™ XL Site-Directed Mutagenesis Kit, Stratagene.

5. pFR-luc, GAL4 reporter construct (Stratagene) consisting of a minimal promoter containing 5 GAL4 binding elements upstream of a TATA box and the firefly luciferase gene.

6. pRLTK (Promega) containing the coding sequence of renilla luciferase, used as transfection standard.

7. ASNH-3 pCDNA3 containing the coding sequence from SEQ ID NO: 5. This sequence is identical to the GeneBank Accession number NM_017902 (Homo sapiens hypoxia-inducible factor 1, alpha subunit inhibitor).

30 ng of pFR-luc reporter plasmid and 10 ng pRLTK internal standard were cotransfected with ASNH-3 and pFA HIF expression plasmids. The amount of transfected pCDNA3 plasmids was kept constant at 60 ng by filling up with pFA-CMV empty cloning vector. Twenty-four hours later, luciferase activity was measured in a lumibox equipped with a Hamamatsu camera (Hamamatsu Photonics, Japan) using after lysis of cells in luciferase buffer.

A luciferase reporter construct in which a 5 X repeat of GAL4 binding sites linked to a TATA box drive firefly luciferase was cotransfected with GAL4-HIF-1 alpha and HIF-2 alpha-TADC fusion plasmids, respectively, and the candidate HIF asparagine-hydroxylase ASNH-3 (Figure 1).

As compared with the vector only control the GAL4-HIF-1 alpha TADC and GAL4 HIF-2 alpha-TADC fusion constructs activated the reporter by a factor of about 300. Cotransfection of ASNH-3 markedly reduced the transactivation activity of GAL4 HIF-1 alpha and HIF-2 alpha fusion protein on the GAL4 responsive reporter gene construct by about 85% (GAL4 HIF-1 alpha TADC) and 80% (GAL4 HIF-2 alpha TADC) (equal amounts of each plasmid were transfected) (Figure 1). A GAL4 HIF-1 alpha TADC mutant, in which alanine was substituted for N813, was by a factor 11.4 more active on the pFR-luc reporter construct than the wild type fusion. This mutant was insensitive to cotransfection with ASNH-3. Concordantly, a GAL4 HIF-2 alpha TADC mutant, in which alanine was substituted for N851, was by a factor 4.6 more active on the pFR-luc reporter construct than the corresponding wild type fusion. Also this mutant was insensitive to cotransfection with ASNH-3.

## EXAMPLE 6

*Tissue-specific expression of ASNH-1, ASNH-2 and ASN-H3*

The qualitative expression pattern of the above mentioned genes in various tissues was determined by real time quantitative polymerase chain reaction. (TaqMan-PCR, Heid et al., Genome Res 6 (10)) on an ABI Prism 7700 sequence detection instrument (Applied Biosystems, Inc.). Therefore, 1 μg of commercially available total RNA from various human tissues (Fa. Clontech) was digested with DNase I and reverse transcribed into cDNA using Superscript-II RT-PCR kit (Gibco, Inc.). 2.5 % of the obtained cDNA was used for each polymerase chain reaction.

The sequences of forward and reverse primers as designed by Primer Express 1.5 Software (Applied Biosystems, Inc.) were 5'-TGCTGCGTTCCATGAAGGT-3' (SEQ ID NO: 7) and 5'-TGCCACAAATCCACCATGAA-3' (SEQ ID NO: 8) for the quantification of ASNH-1, the fluorogenic probe used was 5'-6FAM-CAGAGGATGGCCCACGGGTG-TAMRA-3' (SEQ ID NO: 9). For ASNH-2, the forward and an reverse primer sequences were 5'-ACACACAGTGGCTCACAATGG-3' (SEQ ID NO: 10) and 5'-AAGGGAGCACCTTCCTTCAAG-3' (SEQ ID NO: 11) respectively. The sequence of the fluorogenic probe for the detection of ASNH-2 was 5'-6FAM-CCTCTGGCACCCCAACGTGGC-TAMRA-3' (SEQ ID NO: 12). The forward primer sequence for ASNH-3 was 5'-CATTCCGCGTCTGAGTCAGA-3' (SEQ ID NO: 13), the corresponding reverse primer sequence was 5'-CCACAGGCTCCTCATTCTCAA-3' (SEQ ID NO: 14), sequence of the fluorogenic probe for the quantification of ASNH-3 was 5'-6FAM-CGACCCCCGGGCAGAGGAG-TAMRA-3' (SEQ ID NO: 15). During PCR amplification, 5'-nucleolytic activity of Taq polymerase cleaves the probe separating the 5' reporter fluorescent dye 6FAM (6-carboxy-fluorescein) from the 3' quencher dye TAMRA (6-carboxy-tetramethyl-rhodamine). Because the fluorescence emission will increase in direct proportion to the amount of the specific amplified product, the exponential growth phase of PCR product can be detected and used to determine the initial template concentration. The threshold cycle, Ct, which correlates inversely with the target mRNA level was measured as the cycle number at which the reporter fluorescent emission increases 10 standard deviations above background level. The mRNA levels were corrected for beta-actin mRNA levels to exclude different starting amounts of total RNA and calculated as relative expression using comparative dCt-method (described in TaqMan user guide, Applied Biosystems, Inc.). The expression was calculated using the formula: expr. = $2^{(20\text{-dCt})}$ and is given in arbitrary units.

## EXAMPLE 7

*Proliferation inhibition assay: Antisense oligonucleotides suppress the growth of cancer cell lines*

The cell line used for testing is the human colon cancer cell line HCT116. Cells are cultured in RPMI-1640 with 10-15% fetal calf serum at a concentration of 10,000 cells per ml in a volume of 0.5 ml and kept at 37°C in a 95% air/ 5%$CO_2$ atmosphere.

Phosphorothioate oligoribonucleotides are synthesized on an Applied Biosystems Model 380B DNA synthesizer using phosphoroamidite chemistry. A sequence of 24 bases complementary to the nucleotides at position 1 to 24 of SEQ ID NO: 1 or ID NO: 3 is used as the test oligonucleotide. As a control, another (random) sequence is used: 5'-TCA ACT GAC TAG ATG TAC ATG GAC-3' (SEQ ID NO: 16). Following assembly and deprotection, oligonucleotides are ethanol-precipitated twice, dried, and suspended in phosphate buffered saline at the desired concentration. Purity of the oligonucleotides is tested by capillary gel electrophoresis and ion exchange HPLC. The purified oligonucleotides are added to the culture medium at a concentration of 10 μM once per day for seven days.

The addition of the test oligonucleotide for seven days results in significantly reduced expression of human asparagine-hydroxylase as determined by Western blotting. This effect is not observed with the control oligonucleotide. After 3 to 7 days, the number of cells in the cultures is counted using an automatic cell counter. The number of cells in cultures treated with the test oligonucleotide (expressed as 100%) is compared with the number of cells in cultures treated with the control oligonucleotide. The number of cells in cultures treated with the test oligonucleotide is not more than 30% of control, indicating that the inhibition of human asparagine-hydroxylase has an anti-proliferative effect on cancer cells.

## EXAMPLE 8

*In vivo testing of compounds/target validation*

*Cancer*

1. Acute Mechanistic Assays

*1.1. Reduction in Mitogenic Plasma Hormone Levels*

This non-tumour assay measures the ability of a compound to reduce either the endogenous level of a circulating hormone or the level of hormone produced in response to a biologic stimulus. Rodents are administered test compound (p.o., i.p., i.v., i.m., or s.c.). At a predetermined time after administration of test compound, blood plasma is collected. Plasma is assayed for levels of the hormone of interest. If the normal circulating levels of the hormone are too low and/or variable to provide consistent results, the level of the hormone may be elevated by a pre-treatment with a biologic stimulus (i.e., LHRH may be injected i.m. into mice at a dosage of 30 ng/mouse to induce a burst of testosterone synthesis). The timing of plasma collection would be adjusted to coincide with the peak of the induced hormone response. Compound effects are compared to a vehicle-treated control group. An F-test is performed to determine if the variance is equal or unequal followed by a Student's t-test. Significance is p value $\leq 0.05$ compared to the vehicle control group.

*1.2. Hollow Fiber Mechanism of Action Assay*

Hollow fibers are prepared with desired cell line(s) and implanted intraperitoneally and/or subcutaneously in rodents. Compounds are administered p.o., i.p., i.v., i.m., or s.c. Fibers are harvested in accordance with specific readout assay protocol, these may include assays for gene expression (bDNA, PCR, or Taqman), or a specific biochemical activity (i.e., cAMP levels. Results are analised by Student's t-test or Rank Sum test after the variance between groups is compared by an F-test, with significance at $p \leq 0.05$ as compared to the vehicle control group.

2. Subacute Functional *In Vivo* Assays

*2.1. Reduction in Mass of Hormone Dependent Tissues*

This is another non-tumour assay that measures the ability of a compound to reduce the mass of a hormone dependent tissue (i.e., seminal vesicles in males and uteri in females). Rodents are administered test compound (p.o., i.p., i.v., i.m., or s.c.) according to a predetermined schedule and for a predetermined duration (i.e., 1 week). At termination of the study, animals are weighed, the target organ is excised, any fluid is expressed, and the weight of the organ is recorded. Blood plasma may also be collected. Plasma may be assayed for levels of a hormone of interest or for levels of test agent. Organ weights may be directly compared or they may be normalised for the body weight of the animal. Compound effects are compared to a vehicle-treated control group. An F-test is performed to determine if the variance is equal or unequal followed by a Student's t-test. Significance is p value ≤ 0.05 compared to the vehicle control group.

*2.2. Hollow Fiber Proliferation Assay*

Hollow fibers are prepared with desired cell line(s) and implanted intraperitoneally and/or subcutaneously in rodents. Compounds are administered p.o., i.p., i.v., i.m., or s.c. Fibers are harvested in accordance with specific readout assay protocol. Cell proliferation is determined by measuring a marker of cell number (i.e., MTT or LDH). The cell number and change in cell number from the starting inoculum are analised by Student's t-test or Rank Sum test after the variance between groups is compared by an F-test, with significance at p ≤ 0.05 as compared to the vehicle control group.

*2.3. Angiogenesis Models*

*2.3.1. Corneal Angiogenesis*

Hydron pellets with or without growth factors or cells are implanted into a micropocket surgically created in the rodent cornea. Compound administration may be systemic or local (compound mixed with growth factors in the hydron pellet). Corneas are harvested at 7 days post implantation immediately following intracardiac infusion of colloidal carbon and are fixed in 10% formalin. Readout is qualitative scoring and/or image analysis. Qualitative scores are compared by Rank Sum test. Image analysis data is evaluated by measuring the area of neovascularization (in pixels) and group averages are compared by Student's t-test (2 tail). Significance is p ≤ 0.05 as compared to the growth factor or cells only group.

*2.3.2. Matrigel Angiogenesis*

Matrigel alone or containing compounds, cells or growth factors, is injected subcutaneously. Compounds are administered p.o., i.p., i.v., i.m., or s.c. Matrigel plugs are harvested at predetermined time point(s) and prepared for readout. Readout is an ELISA-based assay for haemoglobin concentration and/or histological examination (i.e. vessel count, special staining for endothelial surface markers: CD31, factor-8). Readouts are analised by Student's t-test, after the variance between groups is compared by an F-test, with significance determined at p ≤ 0.05 as compared to the vehicle control group.

3. Primary Antitumour Efficacy

*3.1. Early Therapy Models*

*3.1.1. Subcutaneous Tumour*

Tumour cells or fragments are implanted subcutaneously on Day 0. Vehicle and/or compounds are administered p.o., i.p., i.v., i.m., or s.c. according to a predetermined schedule starting at a time, usually on Day 1, prior to the ability to measure the tumour burden. Body weights and tumour measurements are recorded 2-3 times weekly. Mean net body and tumour weights are calculated for each data collection day. Anti-tumor efficacy may be initially determined by comparing the size of treated (T) and control (C) tumours on a given day by a Student's t-test, after the variance between groups is compared by an F-test, with significance determined at p ≤ 0.05. The experiment may also be continued past the end of dosing in which case tumour measurements would continue to be recorded to monitor tumour growth delay. Tumour growth delays are expressed as the difference in the median time for the treated and control groups to attain a predetermined size divided by the median time for the control group to attain that size. Growth delays are compared by generating Kaplan-Meier curves from the times for individual tumours to attain the evaluation size. Significance is p ≤ 0.05.

*3.1.2. Intraperitoneal/Intracranial Tumor Models*

Tumour cells are injected intraperitoneally or intracranially on Day 0. Compounds are administered p.o., i.p., i.v., i.m., or s.c. according to a predetermined schedule starting on Day 1. Observations of morbidity and/or mortality are recorded twice daily. Body weights are measured and recorded twice weekly. Morbidity/mortality data is expressed in terms of the median time of survival and the number of long-term survivors is indicated separately. Survival times are used to generate Kaplan-Meier curves. Significance is p ≤ 0.05 by a log-rank test compared to the control group in the experiment.

*3.2. Established Disease Model*

Tumour cells or fragments are implanted subcutaneously and grown to the desired size for treatment to begin. Once at the predetermined size range, mice are randomised into treatment groups. Compounds are administered p.o., i.p., i.v., i.m., or s.c. according to a predetermined schedule. Tumour and body weights are measured and recorded 2-3 times weekly. Mean tumour weights of all groups over days post inoculation are graphed for comparison. An F-test is performed to determine if the variance is equal or unequal followed by a Student's t-test to compare tumour sizes in the treated and control groups at the end of treatment. Significance is $p \leq 0.05$ as compared to the control group. Tumour measurements may be recorded after dosing has stopped to monitor tumour growth delay. Tumour growth delays are expressed as the difference in the median time for the treated and control groups to attain a predetermined size divided by the median time for the control group to attain that size. Growth delays are compared by generating Kaplan-Meier curves from the times for individual tumours to attain the evaluation size. Significance is p value $\leq 0.05$ compared to the vehicle control group.

*3.3. Orthotopic Disease Models*

*3.3.1. Mammary Fat Pad Assay*

Tumour cells or fragments, of mammary adenocarcinoma origin, are implanted directly into a surgically exposed and reflected mammary fat pad in rodents. The fat pad is placed back in its original position and the surgical site is closed. Hormones may also be administered to the rodents to support the growth of the tumours. Compounds are administered p.o., i.p., i.v., i.m., or s.c. according to a predetermined schedule. Tumour and body weights are measured and recorded 2-3 times weekly. Mean tumour weights of all groups over days post inoculation are graphed for comparison. An F-test is performed to determine if the variance is equal or unequal followed by a Student's t-test to compare tumour sizes in the treated and control groups at the end of treatment. Significance is $p \leq 0.05$ as compared to the control group.

Tumour measurements may be recorded after dosing has stopped to monitor tumour growth delay. Tumour growth delays are expressed as the difference in the median time for the treated and control groups to attain a predetermined size divided by the median time for the control group to attain that size. Growth delays are compared by generating Kaplan-Meier curves from the times for individual tumours to attain the evaluation size. Significance is p value $\leq 0.05$ compared to the vehicle control group. In addition, this model provides an opportunity to increase the rate of spontaneous metastasis of this type of tumour. Metastasis can be assessed at termination of the study by counting the number of visible foci per target organ, or measuring the target organ weight. The means of these endpoints are compared by Student's t-test after conducting an F-test, with significance determined at $p \leq 0.05$ compared to the control group in the experiment.

*3.3.2. Intraprostatic Assay*

Tumour cells or fragments, of prostatic adenocarcinoma origin, are implanted directly into a surgically exposed dorsal lobe of the prostate in rodents. The prostate is externalised through an abdominal incision so that the tumour can be implanted specifically in the dorsal lobe while verifying that the implant does not enter the seminal vesicles. The successfully inoculated prostate is replaced in the abdomen and the incisions through the abdomen and skin are closed. Hormones may also be administered to the rodents to support the growth of the tumours. Compounds are administered p.o., i.p., i.v., i.m., or s.c. according to a predetermined schedule. Body weights are measured and recorded 2-3 times weekly. At a predetermined time, the experiment is terminated and the animal is dissected. The size of the primary tumour is measured in three dimensions using either a calliper or an ocular micrometer attached to a dissecting scope. An F-test is performed to determine if the variance is equal or unequal followed by a Student's t-test to compare tumour sizes in the treated and control groups at the end of treatment. Significance is $p \leq 0.05$ as compared to the control group. This model provides an opportunity to increase the rate of spontaneous metastasis of this type of tumour. Metastasis can be assessed at termination of the study by counting the number of visible foci per target organ (i.e., the lungs), or measuring the target organ weight (i.e., the regional lymph nodes). The means of these endpoints are compared by Student's t-test after conducting an F-test, with significance determined at $p \leq 0.05$ compared to the control group in the experiment.

*3.3.3. Intrabronchial Assay*

Tumour cells of pulmonary origin may be implanted intrabronchially by making an incision through the skin and exposing the trachea. The trachea is pierced with the bevelled end of a 25 gauge needle and the tumour cells are inoculated into the main bronchus using a flat-ended 27 gauge needle with a 90° bend. Compounds are administered p.o., i.p., i.v., i.m., or s.c. according to a predetermined schedule. Body weights are measured and recorded 2-3 times weekly. At a predetermined time, the experiment is terminated and the animal is dissected. The size of the primary tumour is measured in three dimensions using either a calliper or an ocular micrometer attached to a dissecting scope. An F-test is performed to determine if the variance is equal or unequal followed by a Student's t-test to compare tumour sizes in the treated and control groups at the end of treatment. Significance is $p \leq 0.05$ as compared to the control group. This model provides an opportunity to increase the rate of spontaneous metastasis of this type of tumour. Metastasis can be assessed at termination of the study by counting the number of visible foci per target organ (i.e., the contralateral lung), or measuring the target organ weight. The means of these endpoints are compared by Student's t-test after conducting an F-test, with significance determined at $p \leq 0.05$ compared to the control group in the experiment.

*3.3.4. Intracecal Assay*

Tumour cells of gastrointestinal origin may be implanted intracecally by making an abdominal incision through the skin and externalising the intestine. Tumour cells are inoculated into the cecal wall without penetrating the lumen of the intestine using a 27 or 30 gauge needle. Compounds are administered p.o., i.p., i.v., i.m., or s.c. according to a predetermined schedule. Body weights are measured and recorded 2-3 times weekly. At a predetermined time, the experiment is terminated and the animal is dissected. The size of the primary tumour is measured in three dimensions using either a calliper or an ocular micrometer attached to a dissecting scope. An F-test is performed to determine if the variance is equal or unequal followed by a Student's t-test to compare tumour sizes in the treated and control groups at the end of treatment. Significance is $p \leq 0.05$ as compared to the control group. This model provides an opportunity to increase the rate of spontaneous metastasis of this type of tumour. Metastasis can be assessed at termination of the study by counting the number of visible foci per target organ (i.e., the liver), or measuring the target organ weight. The means of these endpoints are compared by Student's t-test after conducting an F-test, with significance determined at $p \leq 0.05$ compared to the control group in the experiment.

*Secondary (Metastatic) Antitumour Efficacy*

*3.4. Spontaneous Metastasis*

Tumour cells are inoculated s.c. and the tumours allowed to grow to a predetermined range for spontaneous metastasis studies to the lung or liver. These primary tumours are then excised. Compounds are administered p.o., i.p., i.v., i.m., or s.c. according to a predetermined schedule which may include the period leading up to the excision of the primary tumour to evaluate therapies directed at inhibiting the early stages of tumour metastasis. Observations of morbidity and/or mortality are recorded daily. Body weights are measured and recorded twice weekly. Potential endpoints include survival time, numbers of visible foci per target organ, or target organ weight. When survival time is used as the endpoint the other values are not determined. Survival data is used to generate Kaplan-Meier curves. Significance is $p \leq 0.05$ by a log-rank test compared to the control group in the experiment. The mean number of visible tumour foci, as determined under a dissecting microscope, and the mean target organ weights are compared by Student's t-test after conducting an F-test, with significance determined at $p \leq 0.05$ compared to the control group in the experiment for both of these endpoints.

*3.5. Forced Metastasis*

Tumour cells are injected into the tail vein, portal vein, or the left ventricle of the heart in experimental (forced) lung, liver, and bone metastasis studies, respectively. Compounds are administered p.o., i.p., i.v., i.m., or s.c. according to a predetermined schedule. Observations of morbidity and/or mortality are recorded daily. Body weights are measured and recorded twice weekly. Potential endpoints include survival time, numbers of visible foci per target organ, or target organ weight. When survival time is used as the endpoint the other values are not determined. Survival data is used to generate Kaplan-Meier curves. Significance is $p \leq 0.05$ by a log-rank test compared to the control group in the experiment. The mean number of visible tumor foci, as determined under a dissecting microscope, and the mean target organ weights are compared by Student's t-test after conducting an F-test, with significance at $p \leq 0.05$ compared to the vehicle control group in the experiment for both endpoints.

**EXAMPLE 9**

*In vivo testing of compounds/target validation*

*CNS disorders*

1. Pain

*Acute Pain*

Acute pain is measured on a hot plate mainly in rats. Two variants of hot plate testing are used: In the classical variant animals are put on a hot surface (52 to 56 °C) and the latency time is measured until the animals show nocifensive behavior, such as stepping or foot licking. The other variant is an increasing temperature hot plate where the experimental animals are put on a surface of neutral temperature. Subsequently this surface is slowly but constantly heated until the animals begin to lick a hind paw. The temperature which is reached when hind paw licking begins is a measure for pain threshold.

Compounds are tested against a vehicle treated control group. Substance application is performed at different time points via different application routes (i.v., i.p., p.o., i.t., i.c.v., s.c., intradermal, transdermal) prior to pain testing.

*Persistent Pain*

Persistent pain is measured with the formalin or capsaicin test, mainly in rats. A solution of 1 to 5% formalin or 10 to 100 μg capsaicin is injected into one hind paw of the experimental animal. After formalin or capsaicin application the animals show nocifensive reactions like flinching, licking and biting of the affected paw. The number of nocifensive reactions within a time frame of up to 90 minutes is a measure for intensity of pain.

Compounds are tested against a vehicle treated control group. Substance application is performed at different time points via different application routes (i.v., i.p., p.o., i.t., i.c.v., s.c., intradermal, transdermal) prior to formalin or capsaicin administration.

*Neuropathic Pain*

Neuropathic pain is induced by different variants of unilateral sciatic nerve injury mainly in rats. The operation is performed under anesthesia. The first variant of sciatic nerve injury is produced by placing loosely constrictive ligatures around the common sciatic nerve. The second variant is the tight ligation of about the half of the diameter of the common sciatic nerve. In the next variant, a group of models is used in which tight ligations or transections are made of either the L5 and L6 spinal nerves, or the L% spinal nerve only. The fourth variant involves an axotomy of two of the three terminal branches of the sciatic nerve (tibial and common peroneal nerves) leaving the remaining sural nerve intact whereas the last variant comprises the axotomy of only the tibial branch leaving the sural and common nerves uninjured. Control animals are treated with a sham operation.

Postoperatively, the nerve injured animals develop a chronic mechanical allodynia, cold allodynioa, as well as a thermal hyperalgesia. Mechanical allodynia is measured by means of a pressure transducer (electronic von Frey Anesthesiometer, IITC Inc.-Life Science Instruments, Woodland Hills, SA, USA; Electronic von Frey System, Somedic Sales AB, Hörby, Sweden). Thermal hyperalgesia is measured by means of a radiant heat source (Plantar Test, Ugo Basile, Comerio, Italy), or by means of a cold plate of 5 to 10 °C where the nocifensive reactions of the affected hind paw are counted as a measure of pain intensity. A further test for cold induced pain is the counting of nocifensive reactions, or duration of nocifensive responses after plantar administration of acetone to the affected hind limb. Chronic pain in general is assessed by registering the circadanian rhythms in activity (Surjo and Arndt, Universität zu Köln, Cologne, Germany), and by scoring differences in gait (foot print patterns; FOOTPRINTS program, Klapdor et al., 1997. A low cost method to analise footprint patterns. J. Neurosci. Methods 75, 49-54).

Compounds are tested against sham operated and vehicle treated control groups. Substance application is performed at different time points via different application routes (i.v., i.p., p.o., i.t., i.c.v., s.c., intradermal, transdermal) prior to pain testing.

*Inflammatory Pain*

Inflammatory pain is induced mainly in rats by injection of 0.75 mg carrageenan or complete Freund's adjuvant into one hind paw. The animals develop an edema with mechanical allodynia as well as thermal hyperalgesia. Mechanical allodynia is measured by means of a pressure transducer (electronic von Frey Anesthesiometer, IITC Inc.-Life Science Instruments, Woodland Hills, SA, USA). Thermal hyperalgesia is measured by means of a radiant heat source (Plantar Test, Ugo Basile, Comerio, Italy, Paw thermal stimulator, G. Ozaki, University of California, USA). For edema measurement two methods are being used. In the first method, the animals are sacrificed and the affected hindpaws sectioned and weighed. The second method comprises differences in paw volume by measuring water displacement in a plethysmometer (Ugo Basile, Comerio, Italy).

Compounds are tested against uninflamed as well as vehicle treated control groups. Substance application is performed at different time points via different application routes (i.v., i.p., p.o., i.t., i.c.v., s.c., intradermal, transdermal) prior to pain testing.

*Diabetic Neuropathic Pain*

Rats treated with a single intraperitoneal injection of 50 to 80 mg/kg streptozotocin develop a profound hyperglycemia and mechanical allodynia within 1 to 3 weeks. Mechanical allodynia is measured by means of a pressure transducer (electronic von Frey Anesthesiometer, IITC Inc.-Life Science Instruments, Woodland Hills, SA, USA).

Compounds are tested against diabetic and non-diabetic vehicle treated control groups. Substance application is performed at different time points via different application routes (i.v., i.p., p.o., i.t., i.c.v., s.c., intradermal, transdermal) prior to pain testing.

2. Parkinson's disease

*6-Hydroxydopamine (6-OH-DA) Lesion*

Degeneration of the dopaminergic nigrostriatal and striatopallidal pathways is the central pathological event in Parkinson's disease. This disorder has been mimicked experimentally in rats using single/sequential unilateral stereotaxic injections of 6-OH-DA into the substantia nigra pars compacta (SNC).

Male Wistar rats (Harlan Winkelmann, Germany), weighing 180-200 g at the beginning of the experiment, are used. The rats were maintained in a temperature- and humidity-controlled environment under a 12 h light/dark cycle with free access to food and water when not in experimental sessions. The following in vivo protocols were approved by the governmental authorities. All efforts were made to minimize animal suffering, to reduce the number of animals used, and to utilize alternatives to in vivo techniques.

Animals were administered pargyline on the day of surgery (Sigma, St. Louis, MO, USA; 50 mg/kg i.p.) in order to inhibit metabolism of 6-OHDA by monoamine oxidase and desmethylimipramine HCl (Sigma; 25 mg/kg i.p.) in order to prevent uptake of 6-OHDA by noradrenergic terminals. Thirty minutes later the rats were anesthesized with an i.p. injection of Ketavet (72 mg/kg ketamine; Pharmacia&Upjohn) and Rompun (9.6 mg/kg Xylacin; Bayer) in physiological salt solution (6 ml/kg) and placed in a stereotaxic frame. During surgery, the body temperature was maintained at 37°C with a warming pad. In order to lesion the DA nigrostriatal pathway 4 μl of 0.01%ascorbic acid-saline containing 8 μg of 6-OHDA HBr (Sigma) were injected into the left SNC at a rate of 1 μl/min (3.3 mm anterior, 1.6 mm lateral, -2.1 mm ventral to Knippel and König). The needle was left in place an additional 5 min to allow diffusion to occur.

<u>Stepping Test, rat</u>

Forelimb akinesia was assessed three weeks following lesion placement using a modified stepping test protocol. In brief, the animals were held by the experimenter with one hand fixing the hindlimbs and slightly raising the hind part above the surface. One paw was touching the table, and was then moved slowly sideways (5 s for 1 m), first in the forehand and then in the backhand direction. The number of adjusting steps was counted for both paws in the backhand and forehand direction of movement. The sequence of testing was right paw forehand and backhand adjusting stepping, followed by left paw forehand and backhand directions. The test was repeated three times on three consecutive days, after an initial training period of three days prior to the first testing. Forehand adjusted stepping revealed no consistent differences between lesioned and healthy control animals. Analysis was therefore restricted to backhand adjusted stepping.

<u>Balance Test, rat</u>

Balance adjustments following postural challenge were also measured during the stepping test sessions. The rats were held in the same position as described in the stepping test and, instead of being moved sideways, tilted by the experimenter towards the side of the paw touching the table. This manoeuvre resulted in loss of balance and the ability of the rats to regain balance by forelimb movements was scored on a scale ranging from 0 to 3. Score 0 was given for a normal forelimb placement. When the forelimb movement was delayed but recovery of postural balance detected, score 1 was given. Score 2 represented a clear, yet insufficient, forelimb reaction, as evidenced by muscle contraction, but lack of success in recovering balance, and score 3 was given for no reaction of movement. The test was repeated three times a day on each side for three consecutive days after an initial training period of three days prior to the first testing.

Staircase Test (Paw Reaching), rat

A modified version of the staircase test was used for evaluation of paw reaching behaviour three weeks following primary and secondary lesion placement. Plexiglass test boxes with a central platform and a removable staircase on each side were used. The apparatus is designed such that only the paw on the same side at each staircase can be used, thus providing a measure of independent forelimb use. For each test the animals were left in the test boxes for 15 min. The double staircase was filled with 7 x 3 chow pellets (Precision food pellets, formula: P, purified rodent diet, size 45 mg; Sandown Scientific) on each side. After each test the number of pellets eaten (successfully retrieved pellets) and the number of pellets taken (touched but dropped) for each paw and the success rate (pellets eaten/pellets taken) were counted separately. After three days of food deprivation (12 g per animal per day) the animals were tested for 8 days. Full analysis was conducted only for the last five days.

Rotarod Test, rat

We used a modification of the procedure described by Rozas and Labandeira-Garcia (1997), with a CR-1 Rotamex system (Columbus Instruments, Columbus, OH) comprising an IBM-compatible personal computer, a CIO-24 data acquisition card, a control unit, and a four-lane rotarod unit. The rotarod unit consists of a rotating spindle (diameter 7.3 cm) and individual compartments for each rat. The system software allows preprogramming of session protocols with varying rotational speeds (0-80 rpm). Infrared beams are used to detect when a rat has fallen onto the base grid beneath the rotarod. The system logs the fall as the end of the experiment for that rat, and the total time on the rotarod, as well as the time of the fall and all the set-up parameters, are recorded. The system also allows a weak current to be passed through the base grid, to aid training.

Grip Strength Test, rat

The grip strength meter is a modification of the standard model GSM1054 grip strength meter. Instead of a single grip crossbar connected to a single strain gauge, two grip rods were arranged coaxially and connected to two separate strain gauges. The two strain gauges were connected via a printed circuit chip to a digital readout which quantified the applied strain to each rod in 1 g divisions to a maximum of 2 kg. Two side walls are adjusted approximately 10 cm apart so that when a rat is placed in the apparatus its body remains positioned perpendicular to the grip bars. On each trial the rat is held around the abdomen and lowered into the apparatus so that it grasps the two grip rods, one with each paw, with its back legs standing on the smooth floor of the apparatus. The experimenter then holds the rat by the base of the tail and pulls it gently in a rearward direction. A rat naturally clings to the grip rods to resist the pull until it can no longer hold on and lets go. The applied force at which the rat releases the two rods with each paw is measured separately by the strain gauges.

*MPTP treatment*

The neurotoxin 1-methyl-4-phenyl-1,2,3,6-tetrahydro-pyridine (MPTP) causes degeneration of mesencephalic dopaminergic (DAergic) neurones in rodents, non-human primates, and humans and, in so doing, reproduces many of the symptoms of Parkinson's disease. MPTP leads to a marked decrease in the levels of dopamine and its metabolites, and in the number of dopaminergic terminals in the striatum as well as severe loss of the tyrosine hydroxylase (TH)-immunoreactive cell bodies in the substantia nigra, pars compacta.

In order to obtain severe and long-lasting lesions, and to reduce mortality, animals received single injections of MPTP, and were then tested for severity of lesion 7-10 days later. Successive MPTP injections were administered on days 1, 2 and 3. Animals received application of 4 mg/kg MPTP hydrochloride (Sigma) in saline once daily. All injections were intraperitoneal (i.p.) and the MPTP stock solution was frozen between injections. Animals were decapitated on day 11.

Immunohistology

At the completion of behavioural experiments, all animals were anaesthetized with 3 ml thiopental (1 g/40 ml i.p., Tyrol Pharma). The mice were perfused transcardially with 0.01 M PBS (pH 7.4) for 2 min, followed by 4% paraformaldehyde (Merck) in PBS for 15 min. The brains were removed and placed in 4% paraformaldehyde for 24 h at 4°C. For dehydration they were then transferred to a 20% sucrose (Merck) solution in 0.1 M PBS at 4°C until they sank. The brains were frozen in methylbutan at -20°C for 2 min and stored at -70°C. Using a sledge microtome (mod. 3800-Frigocut, Leica), 25 μm sections were taken from the genu of the corpus callosum (AP 1.7 mm) to the hippocampus (AP 21.8 mm) and from AP 24.16 to AP 26.72. 46 Sections were cut and stored in assorters in 0.25 M Tris buffer (pH 7.4) for immunohistochemistry.

A series of sections was processed for free-floating tyrosine hydroxylase (TH) immunohistochemistry. Following three rinses in 0.1 M PBS, endogenous peroxidase activity was quenched for 10 min in 0.3% $H_2O_2$ ±PBS. After rinsing in PBS, sections were preincubated in 10% normal bovine serum (Sigma) for 5 min as blocking agent and transferred to either primary anti-rat TH rabbit antiserum (dilution 1:2000).

Following overnight incubation at room temperature, sections for TH immunoreactivity were rinsed in PBS (2 x10 min) and incubated in biotinylated anti-rabbit immunoglobulin G raised in goat (dilution 1:200) (Vector) for 90 min, rinsed repeatedly and transferred to Vectastain ABC (Vector) solution for 1 h. 3,.3' -Diaminobenzidine tetrahydrochloride (DAB; Sigma) in 0.1 M PBS, supplemented with 0.005% $H_2O_2$, served as chromogen in the subsequent visualization reaction. Sections were mounted on to gelatin-coated slides, left to dry overnight, counter-stained with hematoxylin dehydrated in ascending alcohol concentrations and cleared in butylacetate. Coverslips were mounted on entellan.

3. Dementia

**The object recognition task**

The object recognition task has been designed to assess the effects of experimental manipulations on the cognitive performance of rodents. A rat is placed in an open field, in which two identical objects are present. The rats inspects both objects during the first trial of the object recognition task. In a second trial, after a retention interval of for example 24 hours, one of the two objects used in the first trial, the 'familiar' object, and a novel object are placed in the open field. The inspection time at each of the objects is registered. The basic measures in the OR task is the time spent by a rat exploring the two object the second trial. Good retention is reflected by higher exploration times towards the novel than the 'familiar' object.

Administration of the putative cognition enhancer prior to the first trial predominantly allows assessment of the effects on acquisition, and eventually on consolidation processes. Administration of the testing compound after the first trial allows to assess the effects on consolidation processes, whereas administration before the second trial allows to measure effects on retrieval processes.

The passive avoidance task

The passive avoidance task assesses memory performance in rats and mice. The inhibitory avoidance apparatus consists of a two-compartment box with a light compartment and a dark compartment. The two compartments are separated by a guillotine door that can be operated by the experimenter. A threshold of 2 cm separates the two compartments when the guillotine door is raised. When the door is open, the illumination in the dark compartment is about 2 lux. The light intensity is about 500 lux at the center of the floor of the light compartment.

Two habituation sessions, one shock session, and a retention session are given, separated by inter-session intervals of 24 hours. In the habituation sessions and the retention session the rat is allowed to explore the apparatus for 300 sec. The rat is placed in the light compartment, facing the wall opposite to the guillotine door. After an accommodation period of 15 sec. the guillotine door is opened so that all parts of the apparatus can be visited freely. Rats normally avoid brightly lit areas and will enter the dark compartment within a few seconds.

In the shock session the guillotine door between the compartments is lowered as soon as the rat has entered the dark compartment with its four paws, and a scrambled 1 mA footshock is administered for 2 sec. The rat is removed from the apparatus and put back into its home cage. The procedure during the retention session is identical to that of the habituation sessions.

The step-through latency, that is the first latency of entering the dark compartment (in sec.) during the retention session is an index of the memory performance of the animal; the longer the latency to enter the dark compartment, the better the retention is. A testing compound in given half an hour before the shock session, together with 1 mg*kg$^{-1}$ scopolamine. Scopolamine impairs the memory performance during the retention session 24 hours later. If the test compound increases the enter latency compared with the scopolamine-treated controls, is likely to possess cognition enhancing potential.

The Morris water escape task

The Morris water escape task measures spatial orientation learning in rodents. It is a test system that has extensively been used to investigate the effects of putative therapeutic on the cognitive functions of rats and mice. The performance of an animal is assessed in a circular water tank with an escape platform that is submerged about 1 cm below the surface of the water. The escape platform is not visible for an animal swimming in the water tank. Abundant extra-maze cues are provided by the furniture in the room, including desks, computer equipment, a second water tank, the presence of the experimenter, and by a radio on a shelf that is playing softly.

The animals receive four trials during five daily acquisition sessions. A trial is started by placing an animal into the pool, facing the wall of the tank. Each of four starting positions in the quadrants north, east, south, and west is used once in a series of four trials; their order is randomized. The escape platform is always in the same position. A trial is terminated as soon as the animal had climbs onto the escape platform or when 90 seconds have elapsed, whichever event occurs first. The animal is allowed to stay on the platform for 30 seconds. Then it is taken from the platform and the next trial is started. If an animal did not find the platform within 90 seconds it is put on the platform by the experimenter and is allowed to stay there for 30 seconds. After the fourth trial of the fifth daily session, an additional trial is given as a probe trial: the platform is removed, and the time the animal spends in the four quadrants is measured for 30 or 60 seconds. In the probe trial, all animals start from the same start position, opposite to the quadrant where the escape platform had been positioned during acquisition.

Four different measures are taken to evaluate the performance of an animal during acquisition training: escape latency, traveled distance, distance to platform, and swimming speed. The following measures are evaluated for the probe trial: time (s) in quadrants and traveled distance (cm) in the four quadrants. The probe trial provides additional information about how well an animal learned the position of the escape platform. If an animal spends more time and swims a longer distance in the quadrant where the platform had been positioned during the acquisition sessions than in any other quadrant, one concludes that the platform position has been learned well.

In order to assess the effects of putative cognition enhancing compounds, rats or mice with specific brain lesions which impair cognitive functions, or animals treated with compounds such as scopolamine or MK-801, which interfere with normal learning, or aged animals which suffer from cognitive deficits, are used.

The T-maze spontaneous alternation task

The T-maze spontaneous alternation task (TeMCAT) assesses the spatial memory performance in mice. The start arm and the two goal arms of the T-maze are provided with guillotine doors which can be operated manually by the experimenter. A mouse is put into the start arm at the beginning of training. The guillotine door is closed. In the first trial, the 'forced trial', either the left or right goal arm is blocked by lowering the guillotine door. After the mouse has been released from the start arm, it will negotiate the maze, eventually enter the open goal arm, and return to the start position, where it will be confined for 5 seconds, by lowering the guillotine door. Then, the animal can choose freely between the left and right goal arm (all guillotine-doors opened) during 14 'free choice' trials. As soon a the mouse has entered one goal arm, the other one is closed. The mouse eventually returns to the start arm and is free to visit whichever go alarm it wants after having been confined to the start arm for 5 seconds. After completion of 14 free choice trials in one session, the animal is removed from the maze. During training, the animal is never handled.

The percent alternations out of 14 trials is calculated. This percentage and the total time needed to complete the first forced trial and the subsequent 14 free choice trials (in s) is analised. Cognitive deficits are usually induced by an injection of scopolamine, 30 min before the start of the training session. Scopolamine reduced the per-cent alternations to chance level, or below. A cognition enhancer, which is always administered before the training session, will at least partially, antagonize the scopolamine-induced reduction in the spontaneous alternation rate.

## Claims

**1.** An isolated polynucleotide being selected from the group consisting of:

a) a polynucleotide encoding an asparagine-hydroxylase polypeptide comprising an amino acid sequence selected from the group constisting of:

i) amino acid sequences which are at least about 28% identical to the amino acid sequence shown in SEQ ID NO: 6; and
ii) the amino acid sequence shown in SEQ ID NO: 6.

b) a polynucleotide comprising the sequence of SEQ ID NO: 5;
c) a polynucleotide which hybridizes under stringent conditions to a polynucleotide specified in (a) and (b) and encodes an asparagine-hydroxylase;
d) a polynucleotide the nucleic acid sequence of which deviates from the nucleic acid sequences specified in (a) to (c) due to the degeneration of the genetic code and encodes an asparagine-hydroxylase; and
e) a polynucleotide, which represents a fragment, derivative or allelic variation of a nucleic acid sequence specified in (a) to (d) and encodes an asparagine-hydroxylase.

**2.** An expression vector containing any polynucleotide sequence of claim 1.

**3.** A host cell containing the expression vector of claim 2.

**4.** A substantially purified asparagine-hydroxylase polypeptide encoded by a polynucleotide of claim 1.

**5.** A method for producing an asparagine-hydroxylase polypeptide, wherein the method comprises the steps of:

a) culturing the host cell of claim 3 under conditions suitable for the expression of the polypeptide; and
b) recovering the asparagine-hydroxylase polypeptide from the host cell culture.

**6.** A method for the detection of a polynucleotide encoding asparagine-hydroxylase polypeptide in a biological sample comprising the following steps:

a) hybridizing any polynucleotide of claim 1 to nucleic acid material of a biological sample, thereby forming a hybridization complex; and
b) detecting said hybridization complex.

**7.** The method of claim 6, wherein before hybridization, the nucleic acid material of the biological sample is amplified.

**8.** A method for the detection of a polynucleotide of claim 1 or an asparagine-hydroxylase polypeptide of claim 4 comprising the steps of:

a) contacting a biological sample with a reagent which specifically interacts with the polynucleotide or the asparagine-hydroxylase polypeptide; and
b) detecting the interaction.

**9.** A diagnostic kit for conducting the method of one of the claims 6 to 8.

**10.** A method of screening for agents which decrease the activity of an asparagine-hydroxylase, comprising the steps of:

contacting a test compound with any asparagine-hydroxylase polypeptide encoded by any polynucleotide of claim 1;
detecting binding of the test compound to the asparagine-hydroxylase polypeptide, wherein a test compound which binds to the polypeptide is identified as a potential therapeutic agent for decreasing the activity of an asparagine-hydroxylase.

**11.** A method of screening for agents which regulate the activity of an asparagine-hydroxylase, comprising the steps of:

a) contacting a test compound with an asparagine-hydroxylase polypeptide encoded by any of the polynucleotides of claim 1; and
b) detecting the asparagine-hydroxylase activity of the polypeptide, wherein a test compound which increases the asparagine-hydroxylase activity is identified as a potential therapeutic agent for increasing the activity of the asparagine-hydroxylase and wherein a test compound which decreases the asparagine-hydroxylase activity of the polypeptide is identified as a potential therapeutic agent for decreasing the activity of the asparagine-hydroxylase.

**12.** A method of screening for agents which decrease the activity of an asparagine-hydroxylase, comprising the steps of:

a) contacting a test compound with any polynucleotide of claim 1; and
b) detecting binding of the test compound to the polynucleotide, wherein a test compound which binds to the polynucleotide is identified as a potential therapeutic agent for decreasing the activity of the asparagine-hydroxylase

**13.** A method of reducing the activity of an asparagine-hydroxylase comprising the step of:

contacting a cell with a reagent which specifically binds to any polynucleotide of claim 1 or an asparagine-hydroxylase polypeptide of claim 4, whereby the activity of the asparagine-hydroxylase is reduced.

**14.** A reagent that modulates the activity of an asparagine-hydroxylase polypeptide or polynucleotide, wherein said reagent is identified by the method of any of the claims 10 to 12.

**15.** A pharmaceutical composition, comprising:

the expression vector of claim 2 or the reagent of claim 14, and
a pharmaceutically acceptable carrier.

**16.** Use of the expression vector of claim 2 or the reagent of claim 14 in the preparation of a medicament for modulating the activity of an asparagine-hydroxylase in a disease.

**17.** Use of claim 16, wherein the disease is a cardiovascular disorder, anaemia, cancer, an inflammatory disease, a fibrotic disorder or a CNS disorder.

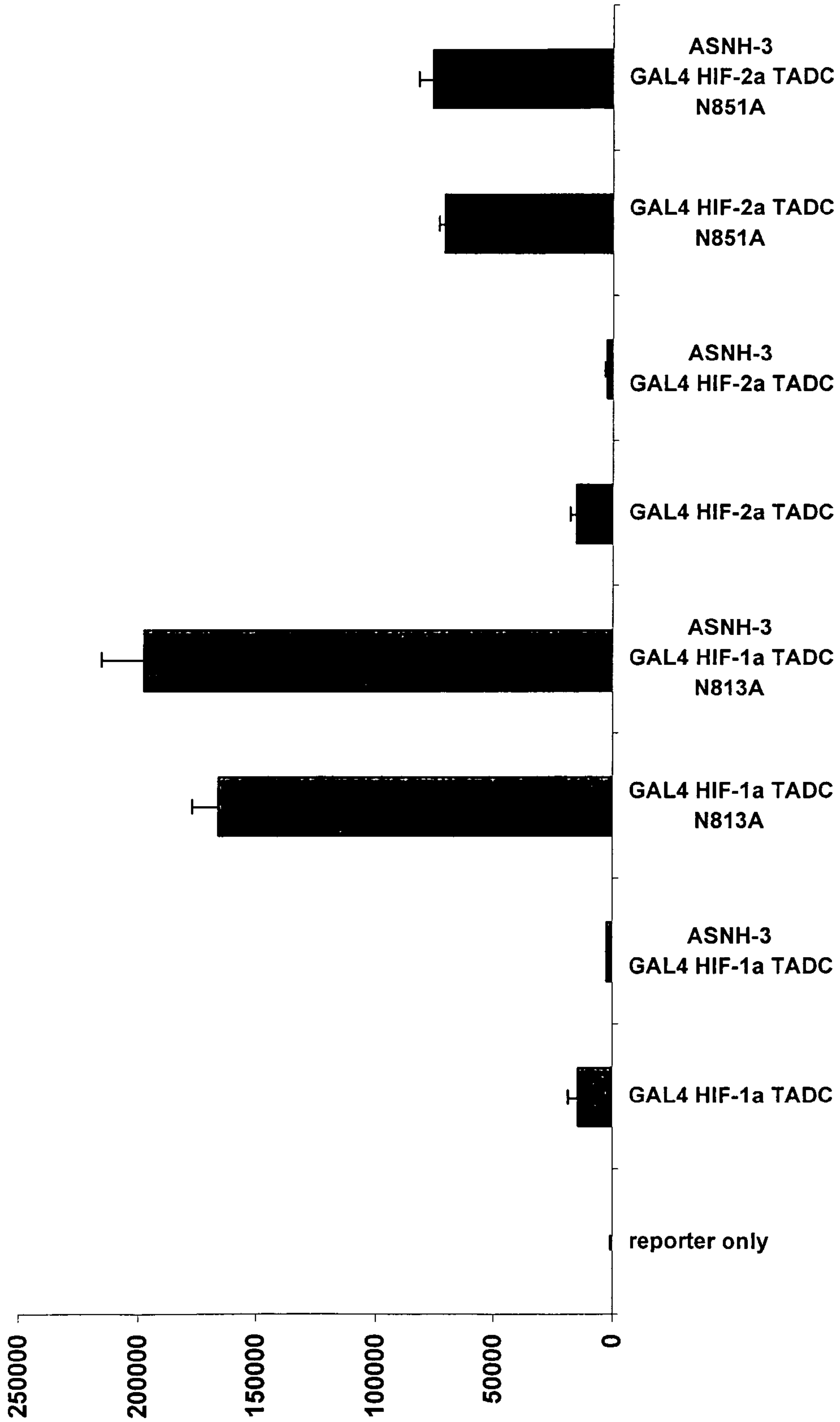
ASNH-3
GAL4 HIF-2a TADC
N851A
GAL4 HIF-2a TADC
N851A
ASNH-3
GAL4 HIF-2a TADC
GAL4 HIF-2a TADC
ASNH-3
GAL4 HIF-1a TADC
N813A
GAL4 HIF-1a TADC
N813A
ASNH-3
GAL4 HIF-1a TADC
GAL4 HIF-1a TADC
reporter only
250000
200000
150000
100000
50000
0

Figure 1

Figure 2

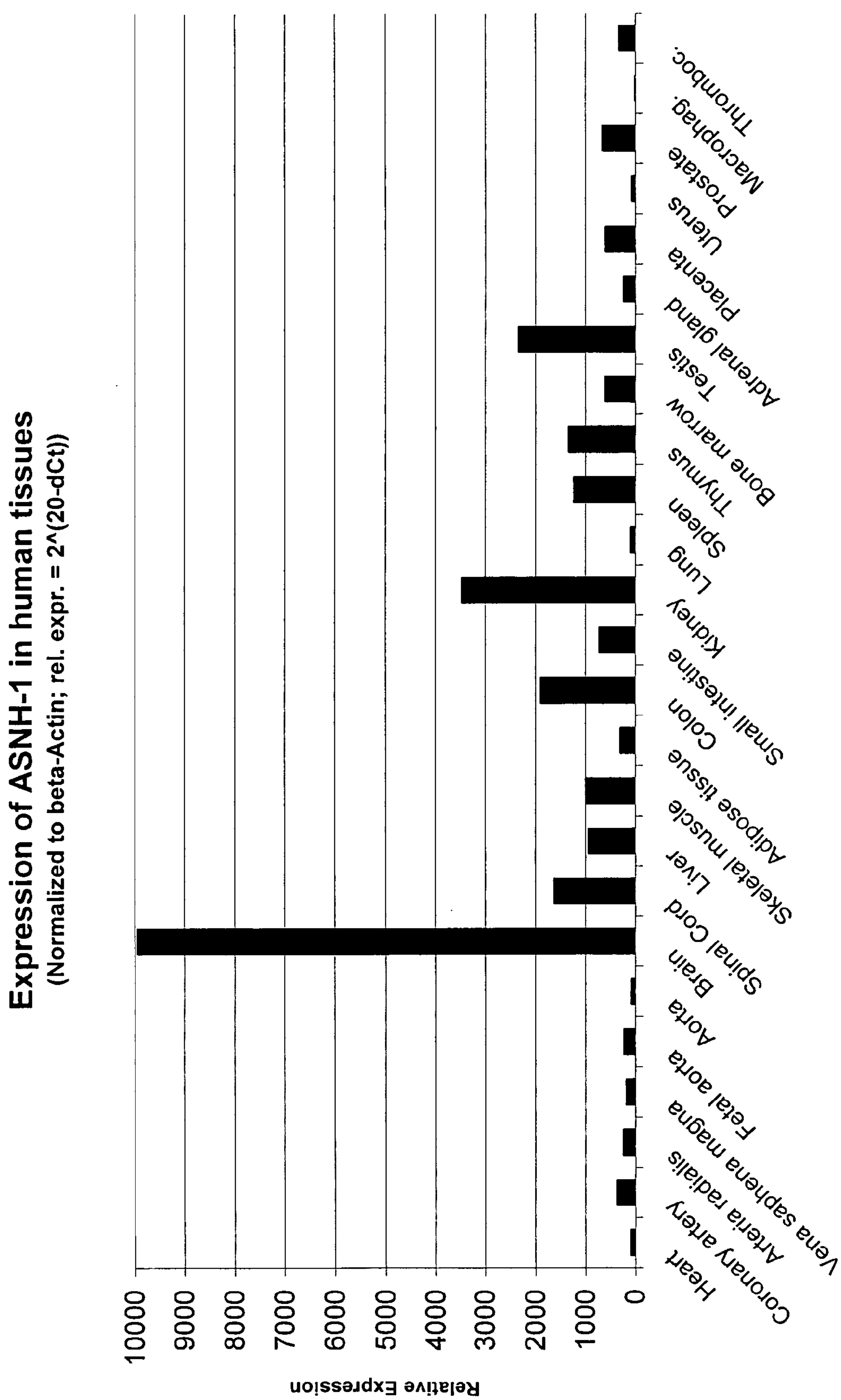

Expression of ASNH-1 in human tissues
(Normalized to beta-Actin; rel. expr. = 2^(20-dCt))
Relative Expression
10000
9000
8000
7000
6000
5000
4000
3000
2000
1000
0
Heart
Coronary artery
Arteria radialis
Vena saphena magna
Fetal aorta
Aorta
Brain
Spinal Cord
Liver
Skeletal muscle
Adipose tissue
Colon
Small intestine
Kidney
Lung
Spleen
Thymus
Bone marrow
Testis
Adrenal gland
Placenta
Uterus
Prostate
Macrophag.
Thromboc.

Figure 3

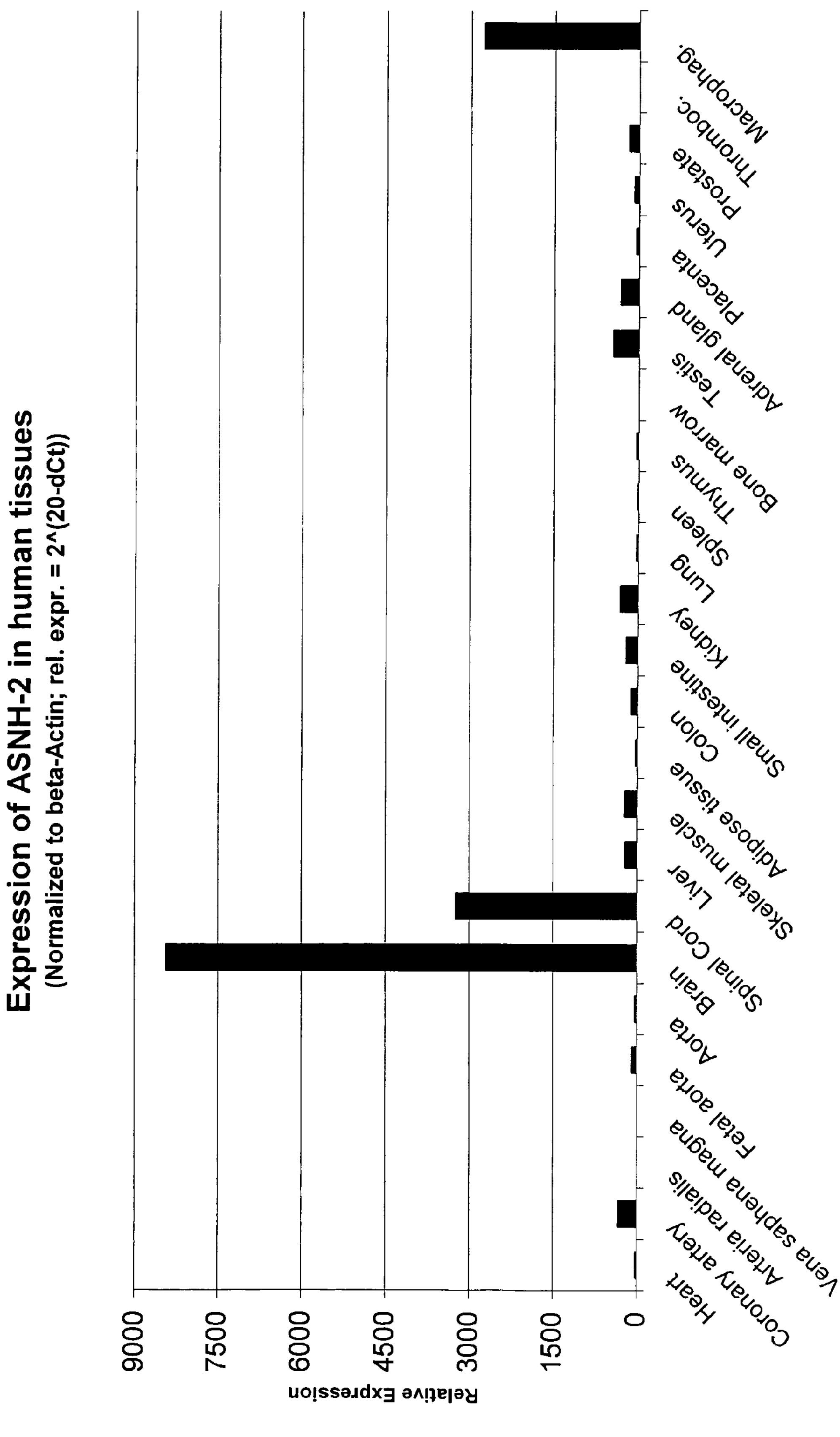
Expression of ASNH-2 in human tissues
(Normalized to beta-Actin; rel. expr. = 2^(20-dCt))
Relative Expression
9000
7500
6000
4500
3000
1500
0
Heart
Coronary artery
Arteria radialis
Vena saphena magna
Fetal aorta
Aorta
Brain
Spinal Cord
Liver
Skeletal muscle
Adipose tissue
Colon
Small intestine
Kidney
Lung
Spleen
Thymus
Bone marrow
Testis
Adrenal gland
Placenta
Uterus
Prostate
Thromboc.
Macrophag.

Figure 4

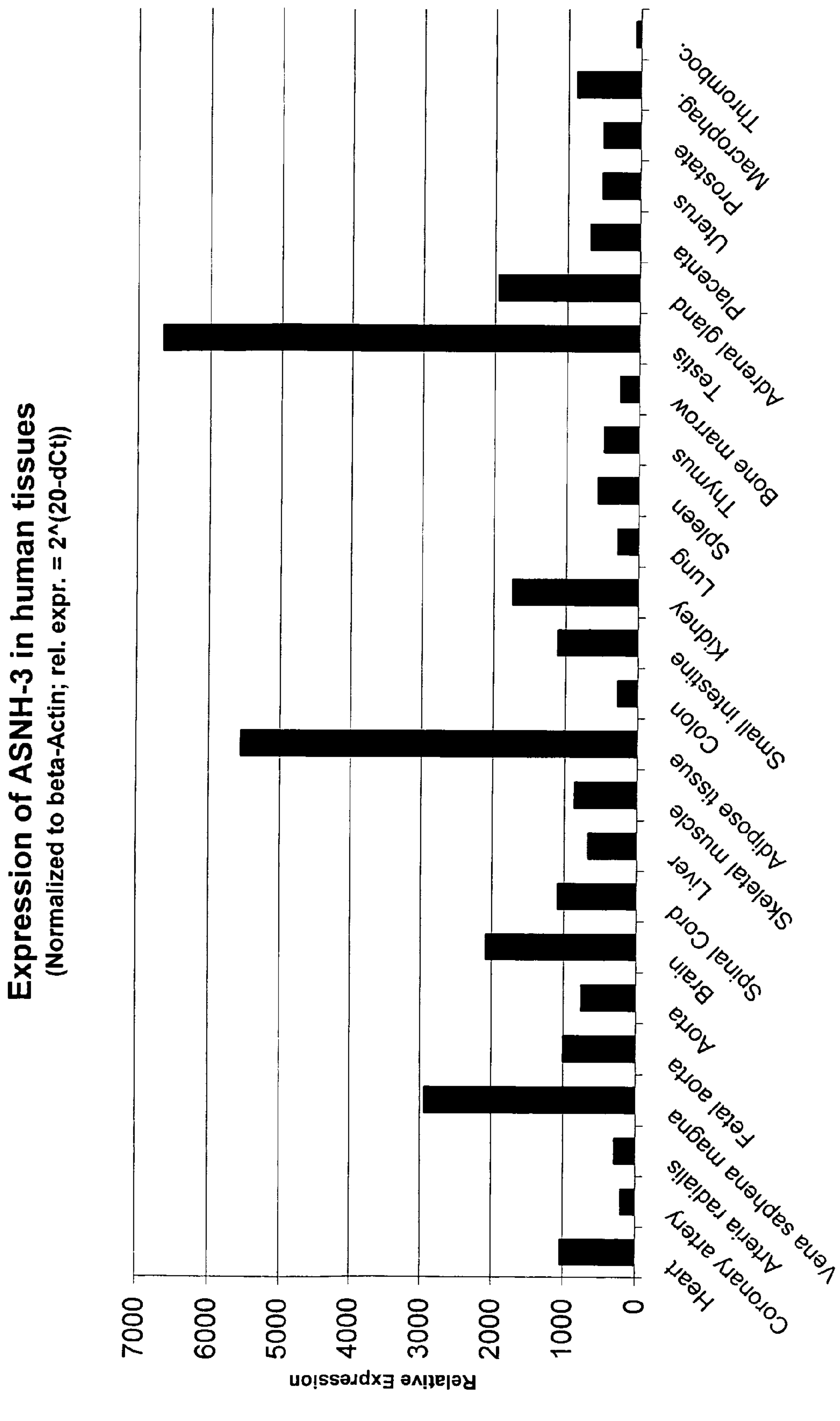
Expression of ASNH-3 in human tissues
(Normalized to beta-Actin; rel. expr. = 2^(20-dCt))
Relative Expression
7000
6000
5000
4000
3000
2000
1000
0
Heart
Coronary artery
Arteria radialis
Vena saphena magna
Fetal aorta
Aorta
Brain
Spinal Cord
Liver
Skeletal muscle
Adipose tissue
Colon
Small intestine
Kidney
Lung
Spleen
Thymus
Bone marrow
Testis
Adrenal gland
Placenta
Uterus
Prostate
Macrophag.
Thromboc.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 2188638 B
- US 5565332 A
- WO 9303151 A
- WO 9413804 A
- US 5641673 A, Haseloff
- EP 321201 A, Gerlach
- US 4843155 A, Chomczynski
- US 5262311 A
- US 5223409 A
- US 5976813 A, Beutel
- US 5283317 A
- WO 9410300 A, Brent
- US 5705151 A


### Non-patent literature cited in the description


- **KORIOTH F ; GIEFFERS C ; FREY, J.** *Gene,* 1994, vol. 150, 395-399
- **WENGER RH ; GASSMANN M.** *Biol Chem.,* 1997, vol. 378 (7), 609-16
- **SEMENZA GL.** *Annu Rev Cell Dev Biol.,* 1999, vol. 15, 551-78
- **ZHU H ; BUNN HF.** *Respir Physiol.,* 1999, vol. 115 (2), 239-47
- **MASSON N ; WILLAM C ; MAXWELL PH ; PUGH CW ; RATCLIFFE PJ.** *EMBO J.,* 17 September 2001, vol. 20 (18), 5197-206
- **LANDO D ; PEET DJ ; WHELAN DA ; GORMAN JJ ; WHITELAW ML.** *Science,* 2002, vol. 295 (5556), 858-61
- **MAHON PC ; HIROTA K ; SEMENZA GL.** *Genes Dev.,* 2001, vol. 15 (20), 2675-86
- **ALTSCHUL et al.** *Bull. Math. Bio.,* 1986, vol. 48, 603
- **HENIKOFF ; HENIKOFF.** *Proc. Natl. Acad. Sci. USA,* 1992, vol. 89, 10915
- **PEARSON ; LIPMAN.** *Proc. Nat'l Acad. Sci. USA,* 1988, vol. 85, 2444
- **PEARSON.** *Meth. Enzymol.,* 1990, vol. 183, 63
- **NEEDLEMAN ; WUNSCH.** *J Mol. Biol.,* 1970, vol. 48, 444
- **SELLERS.** *SIAM J. Appl. Math,* 1974, vol. 26, 787
- **KIVIRIKKO, K. I. ; MYLLYLÄ, T.** *Methods Enzymol.,* 1982, vol. 82, 245-304
- **CUNCLIFFE, C. J. ; FRANKLIN, T. J. ; GASKELL, R. M.** *Biochem. J.,* 1986, vol. 240, 617-619
- **BONNER et al.** *J. Mol. Biol.,* 1973, vol. 81, 123
- **SAMBROOK et al.** *MOLECULAR CLONING: A LABORATORY MANUAL,* 1989, 9.50-9.51
- **BOLTON ; MCCARTHY.** *Proc. Natl. Acad. Sci. U.S.A.,* 1962, vol. 48, 1390
- **SARKAR.** *PCR Methods Applic.,* 1993, vol. 2, 318-322
- **TRIGLIA et al.** *Nucleic Acids Res.,* 1988, vol. 16, 8186
- **LAGERSTROM et al.** *PCR Methods Applic.,* 1991, vol. 1, 111-119
- **PARKER et al.** *Nucleic Acids Res.,* 1991, vol. 19, 3055-3060
- **AUSUBEL et al.** CURRENT PROTOCOLS IN MOLECULAR BIOLOGY. John Wiley & Sons, 1989
- **VAN HEEKE ; SCHUSTER.** *J. Biol. Chem.,* 1989, vol. 264, 5503-5509
- **GRANT et al.** *Methods Enzymol.,* 1987, vol. 153, 516-544
- **TAKAMATSU.** *EMBO J.,* 1987, vol. 6, 307-311
- **CORUZZI et al.** *EMBO J.,* 1984, vol. 3, 1671-1680
- **BROGLIE et al.** *Science,* 1984, vol. 224, 838-843
- **WINTER et al.** *Results Probl. Cell Differ.,* 1991, vol. 17, 85-105
- **HOBBS ; MURRAY.** McGRAW HILL YEARBOOK OF SCIENCE AND TECHNOLOGY. McGraw Hill, 1992, 191-196
- **ENGELHARD et al.** *Proc. Nat. Acad. Sci.,* 1994, vol. 91, 3224-3227
- **LOGAN ; SHENK.** *Proc. Natl. Acad. Sci.,* 1984, vol. 81, 3655-3659
- **SCHARF et al.** *Results Probl. Cell Differ.,* 1994, vol. 20, 125-162
- *ANIMAL CELL CULTURE,* 1986
- **WIGLER et al.** *Cell,* 1977, vol. 11, 223-32
- **LOWY et al.** *Cell,* 1980, vol. 22, 817-23
- **WIGLER et al.** *Proc. Natl. Acad. Sci.,* 1980, vol. 77, 3567-70

- **COLBERE-GARAPIN et al.** *J. Mol. Biol.,* 1981, vol. 150, 1-14
- **HARTMAN ; MULLIGAN.** *Proc. Natl. Acad. Sci.,* 1988, vol. 85, 8047-51
- **RHODES et al.** *Methods Mol. Biol.,* 1995, vol. 55, 121-131
- **HAMPTON et al.** SEROLOGICAL METHODS: A LABORATORY MANUAL. APS Press, 1990
- **MADDOX et al.** *J. Exp. Med.,* 1983, vol. 158, 1211-1216
- **PORATH et al.** *Prot. Exp. Purif.,* 1992, vol. 3, 263-281
- **KROLL et al.** *DNA Cell Biol.,* 1993, vol. 12, 441-453
- **CARUTHERS et al.** *Nucl. Acids Res. Symp. Ser.,* 1980, 215-223
- **HORN et al.** *Nucl. Acids Res. Symp. Ser.,* 1980, 225-232
- **MERRIFIELD.** *J. Am. Chem. Soc.,* 1963, vol. 85, 2149-2154
- **ROBERGE et al.** *Science,* 1995, vol. 269, 202-204
- **CREIGHTON.** PROTEINS: STRUCTURES AND MOLECULAR PRINCIPLES. WH Freeman and Co, 1983
- **KOHLER et al.** *Nature,* 1985, vol. 256, 495-497
- **KOZBOR et al.** *J Immunol. Methods,* 1985, vol. 81, 31-42
- **COTE et al.** *Proc. Natl. Acad. Sci.,* 1983, vol. 80, 2026-2030
- **COLE et al.** *Mol. Cell Biol.,* 1984, vol. 62, 109-120
- **MORRISON et al.** *Proc. Natl. Acad. Sci.,* 1984, vol. 81, 6851-6855
- **NEUBERGER et al.** *Nature,* 1984, vol. 312, 604-608
- **TAKEDA et al.** *Nature,* 1985, vol. 314, 452-454
- **BURTON.** *Proc. Natl. Acad. Sci.,* 1991, vol. 88, 11120-23
- **THIRION et al.** *Eur. J Cancer Prev.,* 1996, vol. 5, 507-11
- **COLOMA ; MORRISON.** *Nat. Biotechnol.,* 1997, vol. 15, 159-63
- **MALLENDER ; VOSS.** *J. Biol. Chem.,* 1994, vol. 269, 199-206
- **VERHAAR et al.** *Int. J. Cancer,* 1995, vol. 61, 497-501
- **NICHOLLS et al.** *J Immunol. Meth.,* 1993, vol. 165, 81-91
- **ORLANDI et al.** *Proc. Natl. Acad. Sci.,* 1989, vol. 86, 3833-3837
- **WINTER et al.** *Nature,* 1991, vol. 349, 293-299
- **BROWN.** *Meth. Mol. Biol.,* 1994, vol. 20, 1-8
- **SONVEAUX.** *Meth. Mol. Biol.,* 1994, vol. 26, 1-72
- **UHLMANN et al.** *Chem. Rev.,* 1990, vol. 90, 543-583
- **GEE et al.** MOLECULAR AND IMMUNOLOGIC APPROACHES. Futura Publishing Co, 1994
- **AGRAWAL et al.** *Trends Biotechnol.,* 1992, vol. 10, 152-158
- **UHLMANN et al.** *Chem. Rev.,* 1990, vol. 90, 543-584
- **UHLMANN et al.** *Tetrahedron. Lett.,* 1987, vol. 215, 3539-3542
- **CECH.** *Science,* 1987, vol. 236, 1532-1539
- **CECH.** *Ann. Rev. Biochem.,* 1990, vol. 59, 543-568
- **CECH.** *Curr. Opin. Struct. Biol.,* 1992, vol. 2, 605-609
- **COUTURE ; STINCHCOMB.** *Trends Genet.,* 1996, vol. 12, 510-515
- **HASELOFF et al.** *Nature,* 1988, vol. 334, 585-591
- CURRENT PROTOCOLS IN MOLECULAR BIOLOGY. John Wiley & Sons, Inc, 1987
- **TEDDER et al.** *Proc. Natl. Acad. Sci. U.S.A.,* 1988, vol. 85, 208-12
- **HEDRICK et al.** *Nature,* vol. 308, 149-53
- **LEE et al.** *Proc. Natl. Acad. Sci. U.S.A.,* 1984, vol. 88, 2825
- **LIANG ; PARDEE.** *Science,* 1992, vol. 257, 967-71
- **DEWITT et al.** *Proc. Natl. Acad. Sci. U.S.A.,* 1993, vol. 90, 6909
- **ERB et al.** *Proc. Natl. Acad. Sci. U.S.A.,* 1994, vol. 91, 11422
- **ZUCKERMANN et al.** *J. Med. Chem.,* 1994, vol. 37, 2678
- **CHO et al.** *Science,* 1993, vol. 261, 1303
- **CARELL et al.** *Angew. Chem. Int. Ed. Engl.,* 1994, vol. 33, 2059
- **CARELL et al.** *Angew. Chem. Int. Ed. Engl.,* vol. 33, 2061
- **GALLOP et al.** *J. Med. Chem.,* 1994, vol. 37, 1233
- **HOUGHTEN.** *BioTechniques,* 1992, vol. 13, 412-421
- **LAM.** *Nature,* 1991, vol. 354, 82-84
- **FODOR.** *Nature,* 1993, vol. 364, 555-556
- **CULL et al.** *Proc. Natl. Acad. Sci. U.S.A.,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 97, 6378-6382
- **FELICI.** *J. Mol. Biol.,* 1991, vol. 222, 301-310
- **JAYAWICKREME et al.** *Proc. Natl. Acad. Sci. U.S.A.,* 1994, vol. 19, 1614-18

- **CHELSKY.** Strategies for Screening Combinatorial Libraries: Novel and Traditional Approaches. *First Annual Conference of The Society for Biomolecular Screening,* 07 November 1995
- **SALMON et al.** *Molecular Diversity,* 1996, vol. 2, 57-63
- **MCCONNELL et al.** *Science,* 1992, vol. 257, 1906-1912
- **SJOLANDER ; URBANICZKY.** *Anal. Chem.,* 1991, vol. 63, 2338-2345
- **SZABO et al.** *Curr. Opin. Struct. Biol.,* 1995, vol. 5, 699-705
- **ZERVOS et al.** *Cell,* 1993, vol. 72, 223-232
- **MADURA et al.** *J. Biol. Chem.,* 1993, vol. 268, 12046-12054
- **BARTEL et al.** *BioTechniques,* 1993, vol. 14, 920-924
- **IWABUCHI et al.** *Oncogene,* 1993, vol. 8, 1693-1696
- **KAULE, G. ; GÜNZLER, V.** *Anal. Biochem.,* 1990, vol. 184, 291-297
- REMINGTON'S PHARMACEUTICAL SCIENCES. Maack Publishing Co,
- **FINDEIS et al.** *Trends in Biotechnol.,* 1993, vol. 11, 202-05
- **CHIOU et al.** GENE THERAPEUTICS: METHODS AND APPLICATIONS OF DIRECT GENE TRANSFER. 1994
- **WU ; WU.** *J. Biol. Chem.,* 1988, vol. 263, 621-24
- **WU et al.** *J Biol. Chem.,* 1994, vol. 269, 542-46
- **ZENKE et al.** *Proc. Natl. Acad. Sci. U.S.A.,* 1990, vol. 87, 3655-59
- **WU et al.** *J. Biol. Chem.,* 1991, vol. 266, 338-42
- **MYERS et al.** *Science,* 1985, vol. 230, 1242
- **COTTON et al.** *Proc. Natl. Acad. Sci. USA,* 1985, vol. 85, 4397-4401
- **CHIRGWIN et al.** *Biochem.,* 1979, vol. 18, 5294-99
- **HEID et al.** TaqMan-PCR. *Genome Res,* vol. 6 (10
- **KLAPDOR et al.** A low cost method to analise footprint patterns. *J. Neurosci. Methods,* 1997, vol. 75, 49-54

SEQUENCE LISTING

<110> Bayer AG

<120> REGULATION OF NOVEL HUMAN ASPARAGINE-HYDROXYLASES

<130> LeA 36154 EP

<160> 16

<170> PatentIn version 3.1

<210> 1

<211> 1487

<212> DNA

<213> Homo sapiens

<220>

<221> misc_feature

<223> ASNH-1

<400> 1

```
ggctcgggct ccggctcggg ctcccgcggc gtgggctggg cagcatgccg ggcggcgggt      60
ccgcggcgcc gggcaggggc cgtccgcggg tccgcgccct cgggcggcgg cgtctcctgg     120
gtcccggcag ccgtccgcgc gcccgggcgc aggtttggtt ttcctaaaca atatcctttc     180
acagggaccg acggcacttg ataatgtgac aaaaaccagc ctcttccacc ccactgcagt     240
gacttttgcc ggaaagtgga gcagtgggca cggccaacct tgtcgttcat caatgccgcc     300
cctggcagta tctgaggatc ggtggcagcc atgcctcccc ctgccccagc cgctccttcc     360
cccccacgct aatctgcatg gtgtgggcgc ccttgggacc cccgaggact gattgtctga     420
ccttgcttca cacgcccagt aaggactccc ccaagatgtc gctcgagtgg ctggtggcct     480
ggagctggtc gctggatggc ctgagggact gcatcgccac cggcatccag tccgtgcggg     540
actgcgacac caccgctgtc atcactgtgg cctgcctcct ggtcctcttc gtgtggtact     600
gttatcacgt gggcagggag cagccccggc cctacgtctc cgtcaactcc ctcatgcagg     660
ctgccgatgc caacgggctg cagaatggct acgtgtactg ccagtcccct gagtgcgtgc     720
```

gctgcaccca caacgagggc ctcaaccaga agctgtacca caacctgcag gagtacgcca 780

agcgctactc ctggtccggc atgggccgca tccacaaggg catccgcgag cagggccggt 840

acctcaacag ccggccctcc atccagaagc ccgaggtctt cttcctgccc gacctgccca 900

ccacgcccta tttctcccgg gacgcacaga aacatgatgt ggaagtgctg gaacggaact 960

tccagaccat cctgtgtgag tttgagaccc tctacaaagc tttctcaaac tgcagcctcc 1020

cgcaaggatg gaaaatgaac agcaccccca gcggggagtg gttcaccttt tacttggtca 1080

atcagggggt ttgtgttccc aggaactgta ggaagtgccc acggacgtac cgcttgctcg 1140

gaagccttcg gacctgtatt gggaacaatg tttttgggaa cgcgtgcatc tctgtgctga 1200

gccctgggac tgtgataacg gagcactatg gacccaccaa catccgcatc cgatgccatt 1260

taggtctgaa aactccaaat ggctgtgagc tggtggtggg gggagagccc cagtgctggg 1320

cagaagggcg ctgccttctc tttgatgact ctttcctgca tgctgcgttc catgaaggtt 1380

cagcagagga tggcccacgg gtggttttca tggtggattt gtggcatcca aacgtcgcag 1440

cggccgaacg gcaggctctt gatttcatct ttgctccggg acgatga 1487

<210> 2

<211> 369

<212> PRT

<213> Homo sapiens

<220>

<221> MISC_FEATURE

<223> ASNH-1

<400> 2

```
Met Val Trp Ala Pro Leu Gly Pro Pro Arg Thr Asp Cys Leu Thr Leu
1               5                   10                  15

Leu His Thr Pro Ser Lys Asp Ser Pro Lys Met Ser Leu Glu Trp Leu
            20                  25                  30

Val Ala Trp Ser Trp Ser Leu Asp Gly Leu Arg Asp Cys Ile Ala Thr
        35                  40                  45

Gly Ile Gln Ser Val Arg Asp Cys Asp Thr Thr Ala Val Ile Thr Val
    50                  55                  60

Ala Cys Leu Leu Val Leu Phe Val Trp Tyr Cys Tyr His Val Gly Arg
65                  70                  75                  80
```

Glu Gln Pro Arg Pro Tyr Val Ser Val Asn Ser Leu Met Gln Ala Ala
85 90 95

Asp Ala Asn Gly Leu Gln Asn Gly Tyr Val Tyr Cys Gln Ser Pro Glu
100 105 110

Cys Val Arg Cys Thr His Asn Glu Gly Leu Asn Gln Lys Leu Tyr His
115 120 125

Asn Leu Gln Glu Tyr Ala Lys Arg Tyr Ser Trp Ser Gly Met Gly Arg
130 135 140

Ile His Lys Gly Ile Arg Glu Gln Gly Arg Tyr Leu Asn Ser Arg Pro
145 150 155 160

Ser Ile Gln Lys Pro Glu Val Phe Phe Leu Pro Asp Leu Pro Thr Thr
165 170 175

Pro Tyr Phe Ser Arg Asp Ala Gln Lys His Asp Val Glu Val Leu Glu
180 185 190

Arg Asn Phe Gln Thr Ile Leu Cys Glu Phe Glu Thr Leu Tyr Lys Ala
195 200 205

Phe Ser Asn Cys Ser Leu Pro Gln Gly Trp Lys Met Asn Ser Thr Pro
210 215 220

Ser Gly Glu Trp Phe Thr Phe Tyr Leu Val Asn Gln Gly Val Cys Val
225 230 235 240

Pro Arg Asn Cys Arg Lys Cys Pro Arg Thr Tyr Arg Leu Leu Gly Ser
245 250 255

Leu Arg Thr Cys Ile Gly Asn Asn Val Phe Gly Asn Ala Cys Ile Ser
260 265 270

Val Leu Ser Pro Gly Thr Val Ile Thr Glu His Tyr Gly Pro Thr Asn
275 280 285

Ile Arg Ile Arg Cys His Leu Gly Leu Lys Thr Pro Asn Gly Cys Glu
290 295 300

Leu Val Val Gly Gly Glu Pro Gln Cys Trp Ala Glu Gly Arg Cys Leu
305 310 315 320

Leu Phe Asp Asp Ser Phe Leu His Ala Ala Phe His Glu Gly Ser Ala
325 330 335

Glu Asp Gly Pro Arg Val Val Phe Met Val Asp Leu Trp His Pro Asn
340 345 350

Val Ala Ala Ala Glu Arg Gln Ala Leu Asp Phe Ile Phe Ala Pro Gly
355 360 365

Arg

<210> 3

<211> 1677

<212> DNA

<213> Homo sapiens

<220>

<221> misc_feature

<223> ASNH-2

<400> 3
cgggactgca ggtccagtgc agggtaggaa ccgctgccca tgggagctag gaggaagcgg 60

ggagagagag cgagcgaaaa gcgggggtgg ggaggaaagg gggagattga ggtgggagag 120

agaagcagat gcgagagaga cggaggctgc tggaaggaga aagaagaggg tgaggaggct 180

gacatgaggg agaggaggaa gaagaggtag aaggagagag aaaggggaga gaacggagag 240

aggagggttg gaggtgcatg aaggagggga gagggagctt cagctgtgga gagagtgacc 300

gcggaaggag agatgagaca gcccagagtg gaatgtggaa gggaaacagt ccagctgggg 360

agccaggggg cagccatgga agggacaggt ggggagctgg ggggacaggg gaactggggt 420

ccggaggacg ccccaggcct cttggccagg gcctccctga tcatgctccc gtggccacta 480

cccctggcct cctcggccct caccttgctc ttcggggccc tcacttccct gttcctctgg 540

tactgctacc gcctgggctc ccaagacatg caggccctag ggctgggag ccgagctggg 600

ggtgttcgtg gtgggcctgt gggatgctcg gaggccggcg ggccaagccc agggggtcct 660

ggggatcccg gggaaggacc taggacggaa ggcctagtga gccggcggct tcgggcctac 720

gcaaggcgct actcctgggc tgggatgggt agagtgaggc gggcagctca gggtggccca 780

ggccctggga gagggccagg ggtcctaggt attcagcgcc caggcctgct tttcctacca 840

gacctgcctt cagcccccctt tgtgccgcgg gacgcccagc ggcacgacgt ggagctcctg 900

gagagcagct tccctgccat tttgcgggac ttcggggctg tgagctggga cttctcaggg 960

actacccctc cgcctcgggg ctggtcccca cctctggccc ccgggtgcta ccagctcctg 1020

ctgtaccaag caggccggtg ccaacccagc aactgccgcc ggtgcccggg ggcctatcgg 1080

gcactgaggg ggcttcgaag ctttatgagt gccaacacct tcggcaatgc cggcttttcc 1140

gttctcctgc ctggggcccg gctcgagggc cgctgtgggc ccaccaatgc ccgggtcaga 1200

tgccatctgg gcctaaagat ccctcctggc tgtgagctgg tggtcggcgg tgagccccag 1260

tgctgggctg aggggcactg tctactggtg gacgactctt ttctacacac agtggctcac 1320

aatggctccc ccgaagatgg gcctcgagtg gtcttcatcg tggacctctg gcaccccaac 1380

gtggcagggg ctgagcgcca ggccctcgac tttgtcttcg ccccagaccc ttgaaggaag 1440

```
gtgctccctt cacacaccca ggctggagag acactgcgct cagggacggc ttgatggtag   1500
ccaggacctc ctctctactg cgggggtggg cgggggcgga ggatgggaac tggctagtga   1560
gcactgaaat ataaattctg aatcctctcc taaaaaaaaa aaaaaaaaaa aaaaaaaaaa   1620
aaaaaaaaaa aaaaaaaaaa aaaaaaaaaa aaaaaaaaaa aaaaaaaaaa aaaaaaa      1677
```

<210> 4

<211> 373

<212> PRT

<213> Homo sapiens

<220>

<221> MISC_FEATURE

<223> ASNH-2

<400> 4

```
Met Arg Gln Pro Arg Val Glu Cys Gly Arg Glu Thr Val Gln Leu Gly
1               5                   10                  15

Ser Gln Gly Ala Ala Met Glu Gly Thr Gly Gly Glu Leu Gly Gly Gln
            20                  25                  30

Gly Asn Trp Gly Pro Glu Asp Ala Pro Gly Leu Leu Ala Arg Ala Ser
        35                  40                  45

Leu Ile Met Leu Pro Trp Pro Leu Pro Leu Ala Ser Ser Ala Leu Thr
    50                  55                  60

Leu Leu Phe Gly Ala Leu Thr Ser Leu Phe Leu Trp Tyr Cys Tyr Arg
65                  70                  75                  80

Leu Gly Ser Gln Asp Met Gln Ala Leu Gly Ala Gly Ser Arg Ala Gly
                85                  90                  95

Gly Val Arg Gly Gly Pro Val Gly Cys Ser Glu Ala Gly Gly Pro Ser
            100                 105                 110

Pro Gly Gly Pro Gly Asp Pro Gly Glu Gly Pro Arg Thr Glu Gly Leu
        115                 120                 125

Val Ser Arg Arg Leu Arg Ala Tyr Ala Arg Arg Tyr Ser Trp Ala Gly
    130                 135                 140

Met Gly Arg Val Arg Arg Ala Ala Gln Gly Gly Pro Gly Pro Gly Arg
145                 150                 155                 160

Gly Pro Gly Val Leu Gly Ile Gln Arg Pro Gly Leu Leu Phe Leu Pro
                165                 170                 175
```

```
Asp Leu Pro Ser Ala Pro Phe Val Pro Arg Asp Ala Gln Arg His Asp
            180                 185                 190

Val Glu Leu Leu Glu Ser Ser Phe Pro Ala Ile Leu Arg Asp Phe Gly
        195                 200                 205

Ala Val Ser Trp Asp Phe Ser Gly Thr Thr Pro Pro Pro Arg Gly Trp
    210                 215                 220

Ser Pro Pro Leu Ala Pro Gly Cys Tyr Gln Leu Leu Leu Tyr Gln Ala
225                 230                 235                 240

Gly Arg Cys Gln Pro Ser Asn Cys Arg Arg Cys Pro Gly Ala Tyr Arg
                245                 250                 255

Ala Leu Arg Gly Leu Arg Ser Phe Met Ser Ala Asn Thr Phe Gly Asn
            260                 265                 270

Ala Gly Phe Ser Val Leu Leu Pro Gly Ala Arg Leu Glu Gly Arg Cys
        275                 280                 285

Gly Pro Thr Asn Ala Arg Val Arg Cys His Leu Gly Leu Lys Ile Pro
    290                 295                 300

Pro Gly Cys Glu Leu Val Val Gly Gly Glu Pro Gln Cys Trp Ala Glu
305                 310                 315                 320

Gly His Cys Leu Leu Val Asp Asp Ser Phe Leu His Thr Val Ala His
                325                 330                 335

Asn Gly Ser Pro Glu Asp Gly Pro Arg Val Val Phe Ile Val Asp Leu
            340                 345                 350

Trp His Pro Asn Val Ala Gly Ala Glu Arg Gln Ala Leu Asp Phe Val
        355                 360                 365

Phe Ala Pro Asp Pro
    370
```

<210> 5

<211> 1050

<212> DNA

<213> Homo sapiens

<220>

<221> misc_feature

<223> ASNH-3

<400> 5
atggcggcga cagcggcgga ggctgtggcc tctggctctg gagagccccg ggaggaggct 60

ggagccctcg gccccgcctg ggatgaatcc cagttgcgca gttatagctt cccgactagg 120

cccattccgc gtctgagtca gagcgacccc cgggcagagg agcttattga gaatgaggag 180

cctgtggtgc tgaccgacac aaatcttgtg tatcctgccc tgaaatggga ccttgaatac 240

ctgcaagaga atattggcaa tggagacttc tctgtgtaca gtgccagcac ccacaagttc 300

ttgtactatg atgagaagaa gatggccaat ttccagaact ttaagccgag gtccaacagg 360

gaagaaatga aatttcatga gttcgttgag aaactgcagg atatacagca gcgaggaggg 420

gaagagaggt tgtatctgca gcaaacgctc aatgacactg tgggcaggaa gattgtcatg 480

gacttcttag gttttaactg gaactggatt aataagcaac agggaaagcg tggctggggg 540

cagcttacct ctaacctgct gctcattggc atggaaggaa atgtgacacc tgctcactat 600

gatgagcagc agaacttttt tgctcagata aaaggttaca aacgatgcat cttattccct 660

ccggatcagt tcgagtgcct ctacccatac cctgttcatc acccatgtga cagacagagc 720

caggtggact ttgacaatcc cgactacgag aggttcccta atttccaaaa tgtggttggt 780

tacgaaacag tggttggccc tggtgatgtt ctttacatcc caatgtactg gtggcatcac 840

atagagtcat tactaaatgg ggggattacc atcactgtga acttctggta taagggggct 900

cccaccccta agagaattga atatcctctc aaagctcatc agaaagtggc cataatgaga 960

aacattgaga agatgcttgg agaggccttg gggaacccac aagaggtggg gcccttgttg 1020

aacacaatga tcaagggccg gtacaactag 1050

<210> 6

<211> 349

<212> PRT

<213> Homo sapiens

<220>

<221> MISC_FEATURE

<223> ASNH-3

<400> 6

Met Ala Ala Thr Ala Ala Glu Ala Val Ala Ser Gly Ser Gly Glu Pro
1 5 10 15

Arg Glu Glu Ala Gly Ala Leu Gly Pro Ala Trp Asp Glu Ser Gln Leu
20 25 30

```
Arg Ser Tyr Ser Phe Pro Thr Arg Pro Ile Pro Arg Leu Ser Gln Ser
        35                  40                  45

Asp Pro Arg Ala Glu Glu Leu Ile Glu Asn Glu Glu Pro Val Val Leu
    50                  55                  60

Thr Asp Thr Asn Leu Val Tyr Pro Ala Leu Lys Trp Asp Leu Glu Tyr
65                  70                  75                  80

Leu Gln Glu Asn Ile Gly Asn Gly Asp Phe Ser Val Tyr Ser Ala Ser
                85                  90                  95

Thr His Lys Phe Leu Tyr Tyr Asp Glu Lys Lys Met Ala Asn Phe Gln
            100                 105                 110

Asn Phe Lys Pro Arg Ser Asn Arg Glu Glu Met Lys Phe His Glu Phe
        115                 120                 125

Val Glu Lys Leu Gln Asp Ile Gln Gln Arg Gly Gly Glu Glu Arg Leu
    130                 135                 140

Tyr Leu Gln Gln Thr Leu Asn Asp Thr Val Gly Arg Lys Ile Val Met
145                 150                 155                 160

Asp Phe Leu Gly Phe Asn Trp Asn Trp Ile Asn Lys Gln Gln Gly Lys
                165                 170                 175

Arg Gly Trp Gly Gln Leu Thr Ser Asn Leu Leu Leu Ile Gly Met Glu
            180                 185                 190

Gly Asn Val Thr Pro Ala His Tyr Asp Glu Gln Gln Asn Phe Phe Ala
        195                 200                 205

Gln Ile Lys Gly Tyr Lys Arg Cys Ile Leu Phe Pro Pro Asp Gln Phe
    210                 215                 220

Glu Cys Leu Tyr Pro Tyr Pro Val His His Pro Cys Asp Arg Gln Ser
225                 230                 235                 240

Gln Val Asp Phe Asp Asn Pro Asp Tyr Glu Arg Phe Pro Asn Phe Gln
                245                 250                 255

Asn Val Val Gly Tyr Glu Thr Val Val Gly Pro Gly Asp Val Leu Tyr
            260                 265                 270

Ile Pro Met Tyr Trp Trp His His Ile Glu Ser Leu Leu Asn Gly Gly
        275                 280                 285

Ile Thr Ile Thr Val Asn Phe Trp Tyr Lys Gly Ala Pro Thr Pro Lys
    290                 295                 300

Arg Ile Glu Tyr Pro Leu Lys Ala His Gln Lys Val Ala Ile Met Arg
305                 310                 315                 320

Asn Ile Glu Lys Met Leu Gly Glu Ala Leu Gly Asn Pro Gln Glu Val
                325                 330                 335

Gly Pro Leu Leu Asn Thr Met Ile Lys Gly Arg Tyr Asn
            340                 345
```

<210> 7

<211> 19

<212> DNA

<213> Artificial sequence

<220>

<223> ASNH-1 primer 1

<400> 7
tgctgcgttc catgaaggt 19

<210> 8

<211> 20

<212> DNA

<213> Artificial sequence

<220>

<223> ASNH-1 primer 2

<400> 8
tgccacaaat ccaccatgaa 20

<210> 9

<211> 25

<212> DNA

<213> Artificial sequence

<220>

<223> ASNH-1 probe

<400> 9
cagaggatgg cccacgggtg tamra 25

<210> 10

<211> 21

<212> DNA

<213> Artificial sequence

<220>

<223> ASNH-2 primer 1

<400> 10
acacacagtg gctcacaatg g 21

<210> 11

<211> 21

<212> DNA

<213> Artificial sequence

<220>

<223> ASNH-2 primer 2

<400> 11
aagggagcac cttccttcaa g 21

<210> 12

<211> 21

<212> DNA

<213> Artificial sequence

<220>

<223> ASNH-2 probe

<400> 12
cctctggcac cccaacgtgg c 21

<210> 13

<211> 20

<212> DNA

<213> Artificial sequence

<220>

<223> ASNH-3 primer 1

<400> 13
cattccgcgt ctgagtcaga 20

<210> 14

<211> 21

<212> DNA

<213> Artificial sequence

<220>

<223> ASNH-3 primer 2

<400> 14
ccacaggctc ctcattctca a 21

<210> 15

<211> 19

<212> DNA

<213> Artificial sequence

<220>

<223> ASNH-3 probe

<400> 15
cgacccccgg gcagaggag 19

<210> 16

<211> 24

<212> DNA

<213> Artificial sequence

<220>

<223> random primer

<400> 16
tcaactgact agatgtacat ggac 24